# EUROPÄISCHE PATENTSCHRIFT

(11) **EP 1 438 692 B1**
(45) Veröffentlichungstag und Bekanntmachung des Hinweises auf die Patenterteilung: **28.12.2005**
(21) Anmeldenummer: 02787347.0
(22) Anmeldetag: 21.10.2002
(51) Int. Cl.: G06F 19/00, A61N 5/10

(54) **VORBEREITEN EINER AUSWAHL VON STEUERGROESSEN FUER EINE EINZUSTELLENDE DOSISVERTEILUNG EINES TECHNISCHEN GERAETS**
PREPARATION OF A SELECTION OF CONTROL VARIABLES FOR A DOSE DISTRIBUTION TO BE REGULATED IN A TECHNICAL APPLIANCE
PREPARATION D'UNE SELECTION DE GRANDEURS DE COMMANDE POUR UNE REPARTITION DES DOSES A REGLER DANS UN APPAREIL INDUSTRIEL

(30) Priorität: 22.10.2001 DE 10151987
(43) Veröffentlichungstag der Anmeldung: 21.07.2004
(73) Patentinhaber: Fraunhofer-Gesellschaft zur Förderung der angewandten Forschung e.V., 80686 München (DE)
(72) Erfinder: TRINKAUS, Hans, 67661 Kaiserslautern (DE); KUEFER, Karl-Heinz, 67685 Weilerbach (DE)
(74) Vertreter: Fricke, Joachim
(86) Internationale Anmeldenummer: PCT/DE2002/003969
(87) Internationale Veröffentlichungsnummer: WO 2003/038725

(56) Entgegenhaltungen:
- H.W. HAMACHER, K.-H. KÜFER: "Inverse radiation therapy planning - a multiple objective optimisation approach" TECHNICAL REPORT (ITWM), Dezember 1999 (1999-12), Seiten 1-14, XP002268597
- ARELLANO A R ET AL: "Clinically oriented inverse planning implementation" PROCEEDINGS OF THE 22ND ANNUAL INTERNATIONAL CONFERENCE OF THE IEEE ENGINEERING IN MEDICINE AND BIOLOGY SOCIETY (CAT. NO.00CH37143), PROCEEDINGS OF THE 22ND ANNUAL INTERNATIONAL CONFERENCE OF THE IEEE ENGINEERING IN MEDICINE AND BIOLOGY SOCIETY, CHIC, Seiten 2071-2074 vol.3, XP010530924 2000, Piscataway, NJ, USA, IEEE, USA ISBN: 0-7803-6465-1
- K.-H. KÜFER, H.W. HAMACHER, TH. BORTFELD: "A multicriteria optimization approach for inverse radiotherapy planning" OPERATIONS RESEARCH PROCEEDINGS, 9. - 12. September 2000, Seiten 3-7, XP009024885 Dresden
- QIUWEN WU ET AL: "IMRT optimization based on the generalized equivalent uniform dose (EUD)" PROCEEDINGS OF THE 22ND ANNUAL INTERNATIONAL CONFERENCE OF THE IEEE ENGINEERING IN MEDICINE AND BIOLOGY SOCIETY (CAT. NO.00CH37143), PROCEEDINGS OF THE 22ND ANNUAL INTERNATIONAL CONFERENCE OF THE IEEE ENGINEERING IN MEDICINE AND BIOLOGY SOCIETY, CHIC, Seiten 710-713 vol.1, XP010530388 2000, Piscataway, NJ, USA, IEEE, USA ISBN: 0-7803-6465-1
- Y. CENSOR: "Mathematical aspects of radiation therapy treatment planning: continuous inversion versus full discretization and optimization versus feasibility" COMPUTATIONAL RADIOLOGY AND IMAGING: THERAPY AND DIAGNOSTICS, Bd. 110, 1999, Seiten 1-12, XP002268598

## Beschreibung

Die Erfindung betrifft eine Strahlendosis-Verteilung auf Targets und Risiken oder eine Therapieplanoptimierung in einer großen Menge von geeigneten Lösungen.

Bei konformierenden Bestrahlungstechniken wird eine räumliche Dosisverteilung angestrebt, die die Wirkung In einem Tumor maximiert und die Nebenwirkungs-Wahrscheinlichkeit minimiert. Die Geschichte der Strahlentherapie zeigt, daß aus der Einführung verbesserter Dosisverteilungen größere Behandlungserfolge resultieren. Im Körperstammbereich, und zum Teil auch im Kopf- und Halsbereich, gibt es jedoch eine Reihe von Zielvolumina, die aufgrund ihrer irregulären, konkaven Gestalt und der unmittelbaren Nachbarschaft zu Risikoorganen selbst mit modernen derzeit im klinischen Bereich verfügbaren Techniken, wie z.B. 3D-Therapieplanung und einer Verwendung von Multileaf-Kollimatoren vor einem Strahlungskopf, noch nicht hochdosiert und zielvolumenkonform bestrahlt werden können, vgl. Bortfeld, "Dosiskonformation In der Tumortherapie mit externer ionisierender Strahlung", Habilitationsschrift, Universität Heidelberg (1995) und Brahme, Treatment Optimization Using Physical and Radiobiological Objective Functions, Radiation Therapy Physics, Herausgeber Smith, Springer, Berlin 1995.

Durch die Entwicklung der inversen Therapieplanung mit offenen Feldern und der intensitätsmodulierten Strahlentherapie (IMRT) hat sich in jüngster Zeit die Situation grundlegend geändert. Eine inverse Planung liefert in einem gewissen Sinn optimierte Dosisverteilungen im Patienten. Bei der konventionellen Therapie werden Feldgewichte, Keilfiltergeometrien und Einstrahl-Richtungen, bei der IMRT intensitätsmodulierte Felder bestimmt. Theoretische Planungsvergleiche und erste klinische Erfahrungen haben gezeigt, daß mit diesen neuen Methoden insbesondere in schwierigen Fällen deutlich verbesserte Dosisverteilungen im Sinne der Konformations-Strahlentherapie erzielbar sind.

Die inverse Therapieplanung verwendet eine Bewertungsfunktion, die jedem Bestrahlungsplan eine einzige Note als Qualitätsmaß zuordnet, vgl. Bortfeld, Stein, Preiser, "Clinically Relevant Intensity Modulation Optimization Using Physical Criteria", proceedings of the XII^{th} ICCR, Salt Lake City 1997, Herausgeber Leavitt und Starkschall, Seiten 1-4 und **Preiser, Bortfeld, Hartwig, Schlegel, Stein,** "A New Program for Inverse Radiotherapy Planning". Seiten 425-428, a.a.O. Es wird dabei ein Bestrahlungsplan in seiner ganzen Komplexität nur durch eine einzige Zahl charakterisiert. Ein Planungssystem sucht dann aus vielen Plänen nach dem Bestrahlungsplan mit der besten Note.

Bei der Planung sind im allgemeinen jedoch mehrere Risikostrukturen mit unterschiedlicher Relevanz für die Lebensqualität des Patienten zu berücksichtigen. Femer steht das Ziel der Applikation einer hohen und homogen verteilten Dosis im Zielvolumen im Widerspruch zur maximalen Schonung der Risikostrukturen. Diese unterschiedlichen, teils widersprüchlichen Ziele werden berücksichtigt, indem Bewertungsfunktionen organweise (d.h. für Zielvolumen und Risikostrukturen) definiert, mit Wichtungsfaktoren skaliert und abschließend aufsummiert werden. Das Ergebnis ist die oben erwähnte (einzige) Note des gesamten Bestrahlungsplans.

In der Praxis liegt ein wesentliches Problem bei dieser Art der inversen Planung in der Festlegung der Wichtungsfaktoren für die relevanten Strukturen. Diese Faktoren sind nicht klinisch interpretierbar, d.h. eine klinische Bewertung bzw. Anordnung von Organen kann nicht einfach in eine vergleichbar angeordnete Liste von Wichtungsfaktoren umgesetzt werden. Die Wichtungen werden daher durch Versuch und Irrtum ermittelt, was eigentlich durch den Einsatz inverser Planung vermieden werden sollte.

Das bedeutet, daß auch bei der inversen Planung im Allgemeinen etliche Optimierungsschritte mit unterschiedlichen Wichtungsfaktoren durchgeführt werden, bis ein akzeptabler Plan gefunden ist. Dies ist äußerst zeitaufwendig und für die bisher relativ geringe klinische Akzeptanz der inversen Planung mitverantwortlich. Ein Beispiel aus der Erfahrung mit der inversen Planung am DKFZ in Heidelberg verdeutlicht den Sachverhalt. Eine Zusammenfassung der Planungszeiten für die letzten 50 behandelten IMRT-Patienten ergab, daß die inverse Planung pro Patient 2 bis 3 Stunden beanspruchte, wobei jeweils 3 bis 15 Pläne bestimmt wurden. Anders ausgedrückt: Die Wichtungsfaktoren wurden 2 bis 14 mal neu angepaßt, jedes Mal wurde neu gerechnet und bewertet, bis ein zufriedenstellendes Ergebnis gefunden war. Mit wachsender Erfahrung auf diesem Gebiet wird man für Standardfälle zweifellos Klassenlösungen für Wichtungsfaktoren und andere Parameter finden, mit denen adäquate Ergebnisse erzielbar sind. Bei komplizierten und neuen Fällen wird man allerdings um die beschriebene zeitwaufwendige Trial and Error Methode nicht umhin kommen.

Ein weiteres Problem der inversen Planung in ihrer heutigen Form liegt im statischen Charakter der Planungsergebnisse. Die optimale Lösung für einen festen, vorab gewählten Satz von Wichtungsfaktoren liefert keine Informationen, ob beispielsweise ein Zugewinn an Dosis im Zielvolumen möglich wäre, wenn man in bestimmten Risikoorganen höhere Dosen zulassen würde.

Beide Probleme, der große Zeitaufwand und der statische Lösungscharakter, führen dazu, daß auch mit inversen Planungsmethoden häufig nicht der für einen Patienten "ideale Plan" gefunden wird. Diese Problematik hat eine Hauptursache, eine **mehrkriterielle Aufgabensteliung -** die Berücksichtigung bzw. Abwägung von Dosisverteilungen in Zielvolumen und Risikostrukturen - wird durch die Einführung künstlicher Gewichtungen in ein einkriterielles Problem transformiert. Diese willkürliche Verengung des Blickes auf eine einzige Zahl nimmt auch einem Arzt die Chance, nach dem Optimierungslauf dynamische Planungsänderungen vorzunehmen.

Idealerweise sollten nach Abschluß des Optimierungsprozesses unabhängige Teil-Bewertungsfunktionen, jeweils zugehörig zum Zielvolumen oder einem Risikoorgan, gleichzeitig betrachtet und modifiziert werden, um dynamisch und interaktiv und insbesondere auch schnell und einfach einen für den Patienten optimalen Bestrahlungsplan ermitteln zu können.

Ein bevorzugter Vergleichswert für die Beurteilung von Strahlungswirkungen ist die biologisch wirksame Equivalent Uniform Dose (EUD), welche mit Hilfe einer organdependenten Konvexkombination des in einer betrachteten Entität maximal erzielten Dosiswertes und des entsprechenden mittleren Dosiswertes beschrieben wird (Max-and-Mean-Modell), vgl. **Thieke, Bortfeld, Küfer,** "Characterization Of Dose Distributions Through The Max & Mean Dose Concept", Submitted to Acta Oncologica (2001).

Die Berechnung optimaler Dosisverteilungen stellt also eine multikriterielle Aufgabe dar, wobei Strahlungsdosen in verschiedenen, voneinander unabhängigen Strukturen - Zielvolumen und Risiken - betrachtet werden. Die zusätzlichen Forderungen nach einer hohen Strahlungsdosis im Ziel und nach möglichst geringen Dosen in den umliegenden Risikoorganen sind inhärent konträr. Daher sollen die Zielsetzungen von dem Optimierungsprozeß entkoppelt betrachtet werden und das inverse Bestrahlungsplanungsproblem bei gegebener Einstrahlgeometrie als multikriterielle Optimierungsaufgabe formuliert werden. Für jede relevante Gewebeentität - ob Zielvolumen oder Risikoorgan - werden voneinander unabhängige Zielsetzungen festgelegt.

Das Planungsproblem für die Strahlentherapie läßt sich in mathematischer Klassifikation - dank der EUD-Modellierung auch mit Hilfe des Max-and-Mean-Modells - als ein multikriterielles lineares Optimierungsproblem darstellen, dessen Zielfunktionen die biologisch wirksamen EUDs in den Risiken und eine Unterbestrahlung des Zielvolumens gleichzeitig zu minimieren suchen.

Aus der Beschreibung des Standes der Technik ist ersichtlich, daß eine Therapieplanung, also eine Vorgabe von Strahlendosen bei der Behandlung von unerwünschtem Gewebe eine erheblich aufwendige und zeitlich sensible Vorgehensweise erfordert, mit einer Vielzahl von Optimierungsschritten, unterschiedlichen Richtungsfaktoren, bis zum Auffinden eines geeigneten Plans, der subjektiv von unterschiedlichen behandelten Personen auch noch unterschiedlich in seiner optimalen Auswahl bewertet werden kann. Jeder Therapieplan besteht dabei aus einer Vielzahl von Bestrahlungen aus unterschiedlichen Richtungen und mit unterschiedlichen Dosen und selbst sogar noch mit einer unterschiedlichen Intensitätsverteilung während eines Strahlungsabschnitts. Die Therapie wird mit einem zeitlichen Abstand wiederholt, so daß unter dem Begriff der Therapie nicht nur eine einmalige Behandlung, sondern auch eine wiederkehrende Behandlung mit größerem zeitlichen Abstand fällt. Für jede Therapie oder Behandlung sind Steuergrößen oder Steuerbefehle im Sinne von Einstellparametem für den Strahlenkopf, das Gerät und viele Umfeldgeräte nötig, so daß die (voreingestellte) Therapie innerhalb von kurzer Zeit automatisiert ablaufen kann.

Die Vorgaben dieser Kenngrößen für konstruktive Objekte oder technische Geräte sollen für die folgende Problemlösung in den Vordergrund gestellt werden. Unter einer Lösung soll bei einem Strahlengerät nicht nur eine gesamte Therapie, sondern auch einzelne zeitliche Abschnitte einer Gesamttherapie verstanden werden, bezogen auf intensitäten und Richtungen, und technisch zusammengesetzt aus einer Vielzahl von Steuergrößen oder Befehlen, die Bezug auf das Strahlengerät als ein Repräsentant von operativen Geräten haben, um beim späteren Durchführen der Therapie die der gefundenen Lösung entsprechenden Strahlendosen im Zielvolumen (als Target oder Tumor) mit einer gewünscht hohen und in den Risikovolumen (wie gefährdete Organe in der Nachbarschaft des Tumors) mit geringstmöglicher Dosis zu erhalten.

Die Erfindung soll das Problem, zeitlich weit auseinander liegende Neuberechnungen zu erfordem vermeiden, und mit einer Vielzahl von Vorberechnungen arbeiten, aber mit der Vielzahl von möglichen "Lösungen" den das Ergebnis festlegenden Bearbeiter nicht überfordern, sondern ihm diese plausibel und visuell so darstellen, dass er scheinbar nur eine ganz geringe Menge von Daten zu vergleichen hat. Gelöst wird die Problemstellung mit der Erfindung nach Anspruch 1 oder 16.

Es wird dabei davon ausgegangen, daß relevante (mathematisch "effiziente") Lösungen vorberechnet oder vorgegeben sind, die mit einer bekannten Strategie berechnet werden können und von der eigentlichen Anwendung zunächst losgelöst sind. Der Einsatz von leistungsfähigen Rechnern erlaubt die Lösung eines multikriteriellen Problems und das Auffinden von geeigneten Einstellungen beispielsweise des Strahlenkopfes und von Intensitätsvertellungen für jede der vorgegebenen Einstrahlrichtungen, um im Ergebnis über eine spätere Behandlung zu denjenigen Dosiswerten zu kommen, die für das Zielvolumen als Target höchstmöglich und für das Risikovolumen oder die mehreren Risikovolumen in der Nachbarschaft des Targets oder im Strahlenweg zum Target mit einem geringstmöglichen, jeweils organabhängigen Maximalwert nur beaufschlagt werden, bevorzugt sogar geringer beaufschlagt werden.

Die Vielzahl von möglichen Lösungen kann gespeichert werden und ist repräsentiert durch ihre jeweilige Wirkung im Sinne von beispielsweise Dosiswerten als Kennwerte für die bei der Rechnung berücksichtigten Gewebevolumen (Target und Risiken). Diese einzelnen Dosen werden nicht zusammengefaßt In einem Skalar, auch nicht mit einzelnen Wichtungsfaktoren, sondern bleiben als Vektor in dem Sinne vorhanden, daß jede Lösung mehrere Kennwerte für die einzelnen Gewebevolumen besitzt.

Die Lösungen setzen voraus, daß diese Dosiswerte innerhalb der organabhängigen Akzeptanzbereiche liegen. Damit steht eine Vielzahl von möglichen Lösungen zur Verfügung, von denen eine dadurch ausgewählt wird, daß zumindest eine Vielzahl, bevorzugt alle gespeicherten Lösungen eine Planungszone begrenzen oder bilden, innerhalb derer eine "optimale Lösung" (Kompromißlösung) visuell einfach und nachvollziehbar aufgefunden werden kann.

Einer aufgefundenen Lösung können Begleitinformationen beigegeben werden, die mit der Änderung innerhalb der Planungsfläche sich mitändern und neben der Planungsfläche dargestellt werden, so zugehörige Visualisierungen von Isodosen (schichtenweise in Transversalschnitten) oder Dosis-Volumen-Relationen, welche kennzeichnen, welcher Anteil von Volumen eines Organs oder Targets welche Strahlungsintensität in Gray [gy] erhält. Diese beiden Darstellungen sind geläufig und können die anhand der Planungszone gefundene Lösung aus mehreren Strahlendosen für Risikoorgane und Targets unmittelbar in ihrer Wirkung bildlich erfaßbar machen.

Die allgemeinere Betrachtung der Erfindung ergibt einen viel breiteren Anwendungsbereich, als denjenigen der Strahlentherapie. Die Erfindung kann mit der beschriebenen Planungszone und einem darin beweglichen (einbeschriebenen) Mehreck als Navigationskörper auch andere Planungsaufgaben vorbereiten, so die Gestaltung von anderen technischen Geräten oder technischen Objekten, wie Motoren von Automobilen oder Flugzeugkonstruktionen, bei denen technische Eigenschaften für diese Objekte bestehen, die oft divergierende Kriterien erfüllen müssen. Für einen Motor eines Automobils oder eines anderen Kraftfahrzeuges können Hubraum, Leistung, Drehzahl und Verbrauch technische Parameter sein, zusätzlich können aber auch wirtschaftliche Parameter mit einfließen, so das Image oder der Nutzen. Diese Parameter sind insgesamt multikriteriell und haben divergierende Entwicklungsrichtungen; wird beispielsweise der Hubraum erhöht, wird der Verbrauch nicht unbedingt gesenkt. Werden hohe Kosten eingespart, kann man nicht mit einem großen Leistungsbereich aufwarten. Für das angesprochene Objekt als Flugzeug sind beispielsweise Parameter wie leichte Bauweise, Materialfestigkeit, Stärke des verwendeten Materials im Wandbereich und Belastungsmöglichkeiten eine technische Seite, während Kosten, Sicherheit und Lebensdauer andere, eher wirtschaftlich orientierte Kriterien sind, die aber auf die technischen Parameter durchaus Einfluß nehmen. Größerer Materialaufwand geht positiv in Richtung einer Lebensdauer und in Richtung der Sicherheit, ist aber nachteilig hinsichtlich der Kosten. Auch die Bauzeit kann Einfluß nehmen, so daß ein Objekt besser geplant werden kann, dessen technische Eigenschaften durch Repräsentanzwerte auf den Achsenabschnitten in der Planungszone dargestellt werden können. Weitere Anwendungen bieten sich bei der Planung von Objekten, wie einer Kläranlage oder einer Müllverbrennungsanlage, bei denen solche technischen Kriterien wie Platz, Durchsatz und Schadstoffbelastung als geeignete Eigenschaften (im Sinne technischer Parameter) Einsatz finden können, während Standortbestimmung, Transportweg und Kosten wirtschaftliche Faktoren sind, die aber die technischen Faktoren mit beeinflussen, bzw. die technischen Faktoren alleine entscheiden nicht über die Gestalt und die Form einer solchen Großanlage. Es lassen sich sogar so schwierige Systeme planen wie der Bau eines (reinen) Fußballstadion, bei dem nicht nur Standort, sondern auch andere technische Realisierungskriterien, wie Verkehrsanbindung, Kostenbeteiligung und Naturschutzgebiete (Flora/Fauna-Habitat-Gebiete) bei der Entscheidungsfindung oder der Planung Berücksichtigung finden. Auch dieses Objekt läßt sich durch technische Eigenschaften beschreiben, die im Rahmen der Planungszone einzelne Achsen erhalten und ein Linienkörper, der dieser Planungszone einbeschrieben ist, erlaubt eine Modifizierung der einzelnen technischen Eigenschaften zum Auffinden einer optimierten Lösung aus einer Lösungsvielfalt, die verfügbar ist.

Auch solche technischen Objekte, die selbst Arbeitsobjekte sind, also nicht nur konstruktive Objekte, sondern auch operative Geräte wie Roboter, Drehmaschinen oder Montageautomaten lassen sich hinsichtlich ihrer technischen Eigenschaften so beschreiben, daß optimierte Planung solcher Geräte realisierbar und überschaubar ist. Eine große Lösungsvielfalt ist dabei nicht mehr zur Unübersichtlichkeit verdammt, sondern kann Basis für eine Navigation des Benutzers sein, der sich in den vielen möglichen Lösungen leichtfüßig zu bewegen vermag und dabei mit leichter Hand optimale Werte für seinen Vorstellungshorizont herausfinden kann. Aus der vorgenommenen Auswahl können die technischen Parameter direkt übernommen und der weiteren Gestaltung (Objektgestaltung oder Bau des konstruktiven Objektes) oder dem weiteren Bau von operativen Geräten zugrunde gelegt werden. In einem speziellen Fall ist auch der Einsatz der Vorbereitung von Pflichtenheften möglich, die aus der beschriebenen Planungsvorbereitung entstehen können.

Durch Verändern eines einbeschriebenen Navigations-Linienkörpers oder eines Navigations-Polygons für den Fall, daß die Planungszone als eine polygonförmige Fläche gebildet ist, stellt das Verfahren ein Mehreck zur Bewertung, dessen Eckwerte von den technischen Eigenschaften oder von Dosiswerten der Risiken und des Targets gebildet werden. Dieses Polygon verläßt die Planungszone nicht und wenn einer der Eckwerte verändert wird, verändert sich das gesamte Polygon innerhalb der Planungszone.

Das beruht darauf, daß nur eine Lösung zusammenhängend dargestellt wird, aber nicht mehr als eine Lösung in einem stationären Zustand der Darstellung dem Betrachter visuell vermittelt wird. Alle Lösungen sind in der Datenbank zwar zum Zugriff verfügbar, werden aber nicht als Lösungen dargestellt. Sie werden lediglich dadurch insgesamt dem Betrachter vermittelt, daß die Planungsfläche die Zusammenfassung aller Lösungen repräsentiert, die hypothetisch möglich wären, wobei die Umgebung der Planungszone nicht ausgewählt werden kann. Die Umgebung kann dabei ein innerhalb des inneren Randes der Planungszone liegender mehreckiger Körper und ein außerhalb des äußeren Randes der Planungszone liegender übriger Bereich sein. Das ist sowohl 2D als auch 3D möglich.

Ein Verändern auf einer der Achsen, beispielsweise ein Verändern auf der Gewichts- oder Risikoachse zu einem höheren Wert, was eigentlich erwünscht ist, verändert die dargestellte Lösung bei einer Therapieplanung so, daß auch die anderen Dosiswerte der Risikoorgane sich verändern. Zumeist wird es so sein, daß nicht alle Wünsche der Maximaldosis für das Target und Minimaldosen für die Risikoorgane gleichzeitig erreicht werden und ein Optimum gesucht werden soll, was subjektiv stark beeinflußt ist. Eine Reduzierung von Strahlendosen auf beispielsweise sehr empfindliche Risikoorgane (Rückenmark oder Sehorgane) kann dazu führen, daß andere Risikoorgane höhere Strahlungsdosen erhalten und auch das Target eine höhere Strahlungsdosis - wie eigentlich erwünscht - erhält. Andere Risikoorgane können dabei weniger empfindlich sein, beispielsweise die Lungenflügel, so daß ihnen eine höhere Strahlungsdosis nicht so sehr schadet, wie empfindlicheren Organen.

Die subjektive Auswahl steht dem Benutzer völlig frei zur Hand, die visuelle Veränderung des einbeschriebenen Linienkörpers erlaubt mit einem Blick die Qualifizierung einer dargestellten Lösung als mehr oder weniger geeignet.

Es ist zu betonen, daß bei der Darstellung nicht mehrere Lösungen miteinander verbunden werden, sondern nur eine Lösung herausgegriffen wird über den zusammenhängenden Navigations-Linienkörper als beispielsweise einbeschriebenes Polygon, dessen Schnittpunkte mit den Achsen die Kennwerte der Strahlendosis des oben beschriebenen Vektors oder Notenvektors oder Strahlendosis-Vektors einer Lösung sind.

Gleiches gilt für die technischen Eigenschaften anderer Geräte oder technischer Objekte (konstruktionsbeeinflußte Entitäten).

Ein Wechsel zu der Darstellung einer anderen Lösung kann bevorzugt fließend geschehen, im Zuge eines Übergangszustandes, bei dem die eine Lösung in die andere Lösung innerhalb der Planungszone übergeblendet wird (Anspruch 8). Die Qualität einer Änderung kann dabei durch den Wechsel des einbeschriebenen Navigationskörpers bewertet werden, der als solches bevorzugt nicht springt.

Jede der mehreren Achsen bildet beispielsweise eine Strahlendosis-Skala für ein Gewebevolumen, so das Zielvolumen und das zumindest eine, bevorzugt zwei und mehrere Risikovolumen. Diese Achsen werden sichtbar auf einer Darstellungseinrichtung, wie einem Sichtschirm aufgetragen und werden im folgenden als Risikoachsen und Targetachse benannt. Es fallen keine zwei Achsen übereinander und nebeneinander liegende Achsen haben zwischen sich einen Winkel größer Null. Benachbarte Achsen verlaufen nicht parallel, um zumindest eine Fläche aufspannen zu können (2D-Betrachtung).

Auf jeder Achse sind Kennwerte angesiedelt und die Gesamtzahl der gespeicherten Lösungen definiert einen jeweiligen Achsenabschnitt auf jeder Achse. Dieser Achsenabschnitt Ist ein Akzeptanzintervall, das ein jeweiliges oberes und unteres Ende besitzt, vorgegeben über die Lösungen und ihre Kennwerte von Strahlendosen bezogen auf die jeweils betroffene Risikoachse oder Targetachse.

Die Akzeptanzintervalle legen die Planungszone fest, wenn ihre jeweiligen Obergrenzen und jeweiligen Untergrenzen miteinander verbunden werden. Zur Veranschaulichung der Planungszone gegenüber dem Umfeld ist diese anders als das Umfeld dargestellt.

Dem Benutzer des Planungswerkzeuges als System oder Verfahren bietet sich damit die Möglichkeit, innerhalb der Planungszone ein Navigations-Polygon oder dem beschriebenen Linienkörper aufzufinden, der ihm persönlich am geeignetsten für eine später stattfindende Therapie oder eine konstruktionsbeeinflußte Entität ist. Mit leichter Hand wird ein unglaubliches Volumen von möglichen Lösungen, hinter dem wiederum eine noch höhere Anzahl von technischen Parametern für die Einstellung des technischen, insbesondere Strahlentherapie-Gerätes, steht auf ihre Folgen im Sinne einer Eigenschaftsverteilung oder einer Dosisverteilung in den Organen und Risiken untersucht, wobei die eigentliche Therapie noch nicht stattfindet, sondern nur deren Planung, die zeitlich (weit) vor einer tatsächlich stattfindenden Therapie liegt und damit nicht unmittelbar mit dieser Therapie gleichzeitig stattfindet (Anspruch 10).

Bestimmte Kennwerte auf den Risikoachsen und der Targetachse können festgehalten oder fixiert werden, um sich vorwiegend mit der Veränderung der noch verbliebenen Kennwerte auf den anderen Achsen zu befassen (Anspruch 12). Zur Vereinfachung dieser Veränderung und zur visuellen Darstellung der Einschränkung der nunmehr noch möglichen Lösungen kann ein (flächiger) Abschnitt der Planungszone herausgenommen werden. Das geschieht dadurch, daß er visuell abgetrennt wird und beispielsweise heller als die noch verbliebene Planungszone gekennzeichnet wird oder mit einer anderen Farbe. Alle Lösungen, die einen schlechteren Kennwert als Dosis besitzen, der auf der Achse liegt, auf der der Kennwert fixiert worden ist, werden gesperrt (Anspruch 12).

Dadurch ergeben sich auch Reduzierungen oder Sperrungen in der übrigen Planungszone, in die sich die gesperrten Lösungen durch die Verbindung der Punkte auf den Risikoachsen und der Targetachse hinein erstrecken.

Es können mehrere Achsen mit zugehörigen Dosis-Kennwerten fixiert werden, so daß auch mehrere Abschnitte aus der gesamten Planungszone herausgenommen werden können.

Die einzelnen aus der weiteren Planung (und der Planungszone) herausgenommenen Abschnitte können wieder hinzugefügt werden (Anspruch 13).

Bei einem (ebenen) Polygon ist das Sperren gleichzusetzen mit einem Fixieren des Scheitelpunktes und alle Kennwerte zwischen Scheitelpunkt und der äußeren Randlinie, also dem außen liegenden Grenzpolygon werden gesperrt.

Die in der Datenbank gespeicherten Lösungen können bevorzugt pareto-optimale Lösungen sein. Es ist zu aufwendig und sinnlos, alle Lösungen darzustellen. Sinnvoll ist es dagegen, ein geeignetes Vertretersystem aus der Menge von (mathematisch) effizienten Lösungen zu berechnen. In der praktischen Umsetzung besteht ein solches Vertretersystem meist aus 100 bis 200 oder mehr Lösungen, die in der Datenbank gespeichert und visuell zur Verfügung gestellt werden können. Eine schnelle Auswahl aus der Vielzahl der Vorschläge in der Datenbank ist trotz ihres Umfanges möglich und kann bei einem Patienten individuell auf ihn zugerichtet oder vorgeplant werden, um seinem Krankheitsverlauf entsprechend und der persönlichen subjektiven Empfindung des entscheidenden Bedieners Rechnung zu tragen.

Gleiches gilt für die Planung der technischen Eigenschaften eines konstruktiven Objekts.

Die Pareto-Optimalität geht davon aus, das multikriterielle Optimierungsproblem so zu lösen, daß die Zielfunktion für eine Strahlentherapie eine vorgegebene (biologisch wirksame) EUD (equivalent uniform dose) in den Risiken und eine Unterbestrahlung des Zielvolumens (des Targets) gleichzeitig zu minimieren sucht, wobei nicht alle Vorgabewerte (die oberen Grenzwerte als Wunschwerte beim Target und die unteren Grenzwerte als Wunschwerte beim Risiko) gleichzeitig erfüllt werden können, aber in einer Gesamtschau bei einer Pareto-Optimalität näherungsweise optimierte Lösungen gefunden werden.

Eine Vielzahl dieser Lösungen bildet vorteilhaft den Inhalt der oben beschriebenen Datenbank, die über das Planungswerkzeug auf nur eine Lösung hin individualisiert werden, die später Anwendung findet.

Ebenso wie bei der therapeutischen Verwendungsmöglichkeit der gefundenen Ergebnisse ist nicht die Planung selbst Gegenstand des Anspruchs, sondern die Ermöglichung einer solchen Planung, durch Bereitstellen eines entsprechenden Werkzeuges, mit dem diese Planung durchgeführt werden kann (Anspruch 1, 16).

**Hamacher und Küfer,** "Inverse Radiation Therapy Planning", Technical Report (ITWM), Dezember 1999, Seiten 1 bis 14 beschreiben zwar bereits einen **multikriteriellen Ansatz** der Bereitstellung von Lösungen einer Therapieplanung, dort insbesondere Seite 7, unten. Nach dortigen Seiten 10,11 und Abstract (a. E.) wird für die Planung aber ein Baum verwendet, der als Suchkriterium dient, unter alleiniger Verwendung von t-Vektoren (dort Seite 11, oben), die in **Küfer, Hamacher, Bortfeld,** "A Multicriteria Optimization Approach for Inverse Radiotherapy Planning", Operations Research Proceedings, 9 Sept. 2000, Dresden, 9. bis 12. September 2000, verbessert werden, dort Seiten 6,7, insbesondere Seite 6, Mitte. Zwei parallele Skalen dienen neben textlich dargestellten zwei Vektoren als Darstellungs- und Bewertungshilfe für Dosislevel der Organe (Risk und Target).

Ausführungsbeispiele erläutern und ergänzen die Erfindung.
- **Figur 1**: ist eine schematische Darstellung eines technischen Gerätes, hier als Strahlungsgerät 10,11 mit einer Achse 100, um die der bewegliche Teil des Gerätes mit dem Strahlungskopf 12 schwenkbar ist, wobei Schwenkwinkel α eingestellt werden. Abgebildet ist auch die Speichereinheit 5 mit einem Hauptspeicher 1, der eine Datenbank beinhaltet.
- **Figur 2**: ist eine schematische Ansicht eines Transversalschnittes eines Patienten mit drei Risikoorganen h₁,h₂ (Lungenflügel) und h₃ (Rückenmark) sowie einem Zielorgan T als ein Tumor.
- **Figur 2a bis Figur 2e**: symbolisieren Intensitätsverteilungen, wie sie durch eine Multileaf-Struktur vor dem Strahlungskopf 12 von Figur 1 erhalten werden. Die Figuren sind gegenüber der Figur 2 so angeordnet, daß eine jeweilige Intensitätsverteilung an dem in Figur 2 eingezeichneten Schwenkwinkel α gemäß Figur 1 angewendet wird, so die Figur 2a mit der dort gezeigten Intensitätsverteilung aus dem Winkel α₁, die Figur 2b aus dem Winkel α₂, usw.
- **Figur 3**: veranschaulicht schematisch die Multileaf-Einstellungen von streifenförmigen Blättem 13,14, die einen Zwischenraum 12a des Kopfes 12 freigeben, durch den eine in ihrer Intensität auf eine bestimmte Geometrie oder Kontur verteilte Strahlung austritt.
- **Figur 3a**: ist eine Veranschaulichung von unterschiedlichen Einstrahlrichtungen α bei in ihrer Intensität modulierten Strahlungseinflüssen aus einer jeweiligen Richtung, bezogen auf das Ziel T.
- **Figur 4**: ist eine Blockschaltbildstruktur der Rechnerkonfiguration 20 zur Berechnung von Lösungen für die Datenbank 1.
- **Figur 5**: veranschaulicht eine zweidimensionale Darstellung einer Planungszone 40 mit allen darin verwendeten grafischen Komponenten, zur Vorgabe von Steuerwerten oder Steuergrößen, wie solchen Steuergrößen zur Einstellung der Multileaf-Struktur von Figur 3.
- **Figur 6**: soll eine (farbige) Übersicht über alle verwendeten grafischen Strukturen geben, die in den folgenden Figuren in einem Beispiel einer Planung einer Therapie verwendet werden, die nach ihrer Off-line-Festlegung später zum Gegenstand der Behandlung werden kann. Veranschaulicht ist ein Start einer Planungssitzung, bei der die Planungsfläche 40 mit einigen zusätzlichen Elementen belegt ist, zur Erläuterung ihrer Funktion und zur Entlastung der Bezugszeichen in Figur 5.
- **Figur 7**: ist die Planungsfläche 40 von Figur 6 mit einem geänderten Polygonzug P2 als Naviagtions-Polygon oder Navigations-Linienkörper innerhalb der hier 2D dargestellten Planungsfläche 40.
- **Figur 8**: ist ein weiterer Schritt mit einer weiter reduzierten Dosis gegenüber Figur 7 hinsichtlich der Risikoachse h₃ auf 25gy.
- **Figur 9**: ist eine Blockierung oder Festlegung des zuvor genannten Wertes von 25 gy auf der h₃-Achse und ein Ausblenden zumindest des linken Abschnitts oberhalb des Kennwertes 53.
- **Figur 10**: ist eine Veränderung des oberen Kennwertes 50c von Figur 9 zum Wert 50 mit 75gy auf der Targetachse T.
- **Figur 11**: ist eine Darstellung eines Volumen-Histogramms als 3D-Repräsentanz auf flächiger Darstellung.

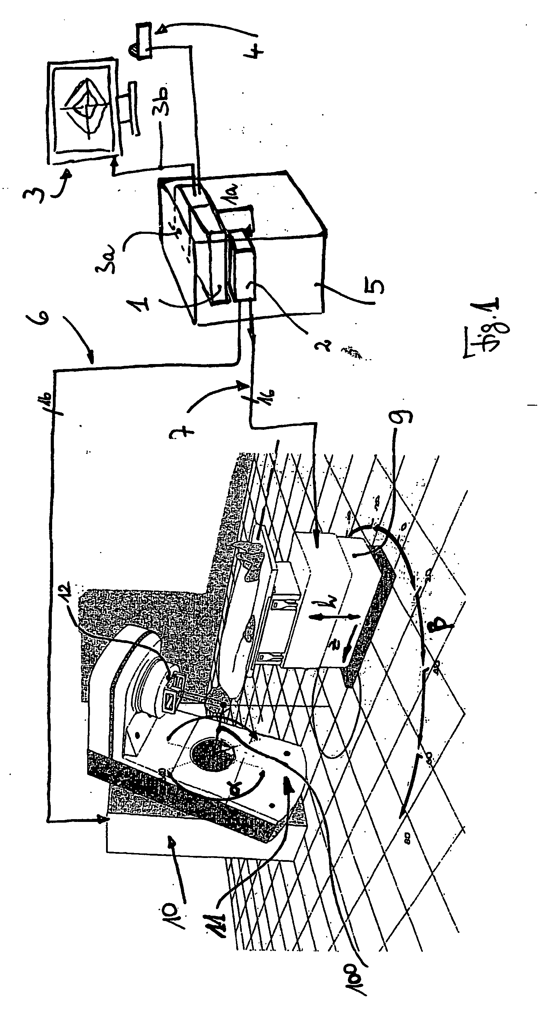

Die Planung für eine hier als Beispiel herausgegriffenen Therapie, die nicht unmittelbar mit der Planung zusammenhängt, sondern davon zeitlich und räumlich beabstandet vor sich geht, soll im Übersichtsschaubild der **Figur 1** veranschaulicht werden. Ein Sichtschirm 3 bildet grafisch Lösungen ab, die dem Benutzer oder Bediener, meist ein erfahrener Arzt, eine Möglichkeit der visuellen Darstellung bietet. Dargestellt werden vorausberechnete Lösungen, die in einem Speicher in einer Datenbank 1 in dem Steuersystem 5 vorgehalten werden. Diese Datenbank-Lösungen werden über ein grafisches Interface 3a über die Steuerleitung 3b auf den Sichtschirm 3 übertragen. Eine Steuereinrichtung 4 zur Beeinflussung und zur Ausführung von interaktiven Eingaben ist in Reichweite des Benutzers nahe dem Sichtschirm 3 placiert.

Ist eine Lösung aus der Datenbank - wobei die Lösung eine Zusammensetzung einer Vielzahl von Steuergrößen eines beispielsweise Strahlentherapiegerätes ist - gefunden, so wird diese über die Steuerleitung 1 a einem weiteren Interface 2 im Rahmen des Steuersystems 5 übergeben, wo sie zwischengespeichert werden kann. Sie kann anschließend über Steuerleitungen 6 digital an eine weitere Steuer- und Überwachungseinrichtung 10 des Strahlentherapiegerätes weitergeleitet werden. Sie kann ebenfalls an eine hebbare oder drehbare Stützeinrichtung 9 über eine weitere Datenleitung 7 übertragen werden. Die Stützeinrichtung 9, für die Aufnahme eines Patienten, ist in einem Winkel β (beta) drehbar und in einer Höhe h und Längsrichtung z verfahrbar.

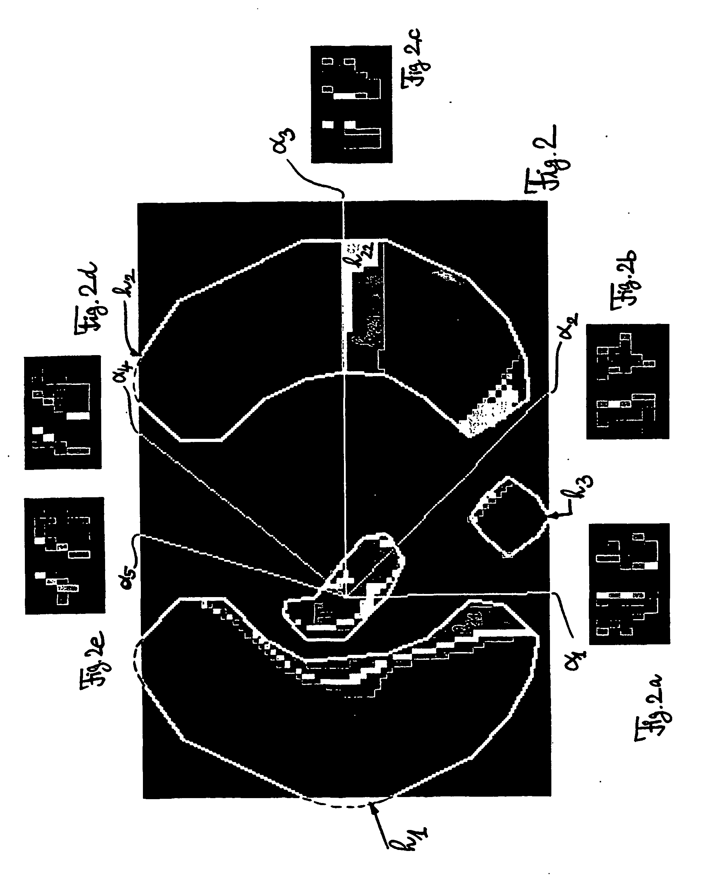

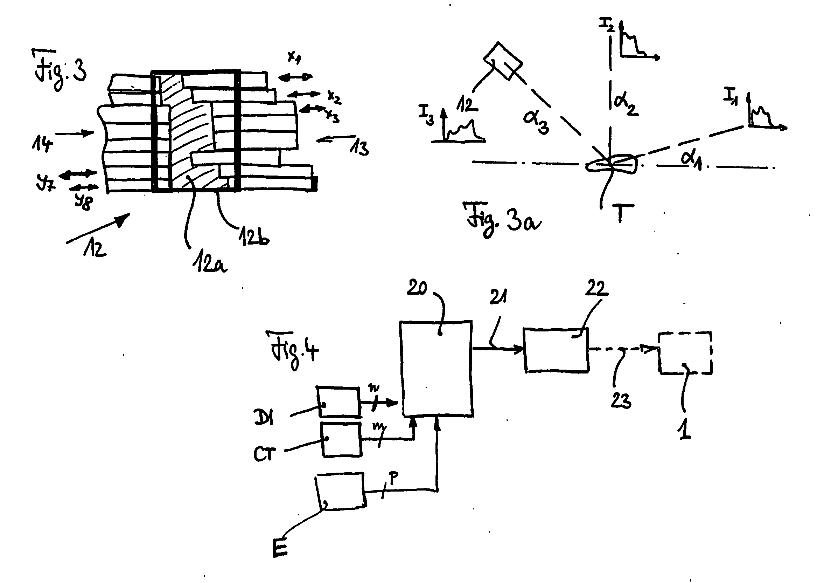

Das Strahlentherapiegerät besteht aus einem festen Systemteil 10 und einem demgegenüber um die Achse 100 schwenkbaren zweiten Teil 11, der einen Strahlenkopf 12 trägt, der auf einen hypothetisch als auf dem Tragtisch 9 liegenden Patienten ausgerichtet ist. Mit den beschriebenen Bewegungen kann der Strahlenkopf in mehreren Richtungen α eingestellt werden, wozu ihm entsprechenden Steuergrößen übermittelt werden. Eine Strahlendosis des Strahlenkopfes 12 wird mit dem im folgenden näher beschriebenen Multileaf-Kollimator gemäß Figur 3 erläutert. Zusätzlich zu einer Drehung um die Achse α kann auch eine Drehung um die Achse β und eine Verlagerung der Tischhöhe und auch der Tischposition erfolgen, gesteuert von den Steuergrößen oder Steuer-Befehlen, die als Sollwerte aus der Datenbank 1 des Steuergerätes 5 übernommen werden.

Sind alle Kennwerte für eine Therapiesitzung übertragen und in dem Gerät eingestellt, kann die Therapie beginnen, die hier aber nicht Gegenstand sein soll.

Die in der Datenbank gespeicherten Lösungen sind im Beispiel pareto-optimale Lösungen, die ein Abschwächen einer optimalen Lösung sind, um in einem multikriteriellen System überhaupt Lösungen zu finden, welche bei widersprüchlichen Zielen akzeptabel sind.

Eine pareto-optimale Lösung ist ein Kompromiß zwischen einer Unterbestrahlung und einer Überbestrahlung, wobei jeweils Berücksichtigung findet, ob es sich um ein Risikoorgan oder das Target handelt. Beim ersteren sind maximale Werte nicht zu überschreiten, beim letzteren sind minimale Werte nicht zu unterschreiten und bestenfalls maximal vorgegebene Sollwerte zu erhalten, ohne die Risikoorgane über die Grenzwerte hinaus zu belasten.

Im folgenden soll von drei Risikoorganen ausgegangen werden, bei einem Zielvolumen T, wie in der **Figur 2** in einem Transversalschnitt veranschaulicht. Es soll erneut betont werden, daß kein therapeutisches Verfahren hier beschrieben und beansprucht wird, aber die Verbindung der hier beschriebenen Erfindung zur Ermöglichung eines therapeutischen Verfahrens dadurch deutlich gemacht werden soll, daß die Ergebnisse, die erzielt werden, in einen funktionellen technischen Zusammenhang gestellt werden, was eine mit einer kurzen Beschreibung sowohl des therapeutischen Verfahrens wie auch eine detaillierte Beschreibung der Vorarbeit erreicht werden soll.

Dazu ist in der Figur 2 schematisch der Transversalschnitt in Höhe von beispielsweise der Niere eines Patienten gezeigt, mit zwei Lungenflügeln h₁, h₂ als zwei Risikoorgane und einem Rückenmark h₃ als einem erhöht gefährdeten Risikoorgan. Ein Tumor T ist als Zielobjekt ausgebildet und liegt zwischen den Lungenflügeln, etwa auf der Höhe der Leber. Es sind diesbezüglich fünf Richtungen α₁,α₂,α₃,α₄ und α₅ eingezeichnet, die fünf Richtungen symbolisieren, aus welchen eine Einstrahlung mit dem Kopf 12 bei entsprechender Anpassung des schwenkbaren Teils 11, so daß die einstellbaren Winkel α aus Figur 1 denjenigen Winkels α₁ bis α₅ von Figur 2 entsprechen können. Eine entsprechende Verlagerung der Stützeinrichtung 9 in Richtung z ist dazu weiterhin möglich, ggf. auch eine Schwenkung des Tisches um den Winkel β, um diejenige Position mit dem Strahlenkopf 12 zu erreichen, die im Schnitt in Figur 2 dargestellt ist.

Die Wirkung eines in seiner Strahlungsintensität modulierten Kopfes 12 mit einer beliebigen Strahlung, wie Photonen, Elektronen, schwere Ionen oder Protonen, veranschaulichen die Schemabilder der **Figuren 2a bis 2e**. Dazu ist Figur 3 heranzuziehen, die eine mit mehreren Streifen versehene Kopfstruktur 12 zeigt, wobei das wirksame Kopffenster 12a innerhalb eines Rahmens 12b von lateral verschiebbaren Streifenstrukturen 13,14 eingestellt wird. Dabei ist sowohl die Geometrie, wie auch die Lage des Fensters 12a veränderbar, wenn die entsprechenden Längsbewegungen oder Längsstellungen als Steuerparameter beachtet werden. Diese Längsstellung x₁ bis x₃ sowie y₇ und y₈ als jeweilige Repräsentariten der Streifen 13 und 14 sind Steuergrößen, die einzustellen sind und Intensitätsverteilungen in Rasterstruktur ergeben, wie sie von den Figuren 2a bis 2e veranschaulicht werden. Wird eine erneute Strahlung abgegeben, bei unterschiedlich eingestellten Steuerblättem 13,14, können auch Intensitätsverteilungen mit unterschiedlichen Intensitäten im Rahmen der Raster erhalten werden. Das in den Figuren 2a bis 2e jeweils schwarz abgebildete Fenster zwischen den intensitätsmodulierten Rasterfeldem ist entsprechend demjenigen Fenster 12a von Figur 3 zu sehen.

Nochmals verdeutlicht und schematisch herausgegriffen ist an Figur 3a die Einstellung von unterschiedlichen Winkeln α₁ bis α₃ bei jeweils intensitätsmodulierten Strahlungsfeldem, wie sie aus der Kombination der Figuren 1 und 2 erhalten werden.

Figur 3a veranschaulicht dabei das Ziel T, wie in Figur 2 gezeigt. Drei unterschiedliche Zielrichtungen α₁, α₂ und α₃ sind eingezeichnet und aus einer jeweiligen Zielrichtung ist ein vorgegebenes Strahlenprofil mit einer Intensitätsverteilung I₁,I₂ und I₃ zu sehen, betreffend den Kopf 12, der zuvor in Figur 3 und in Figur 1 erläutert worden ist. Das Ziel oder Target, das als Zielvolumen mit einer sehr hohen Strahlendosis (Kurativdosis genannt) beaufschlagt werden soll, um eine hohe Kontrollwahrscheinlichkeit der klonogenen Zellen zu erreichen, entspricht dem von Figur 2 und soll hier in der Behandlung aber ebenfalls nicht beansprucht werden, sondern in seiner Bedeutung und der Wirkung nach der Einsetzung des Ergebnisses der hier beschriebenen Lösungssuche veranschaulicht werden.

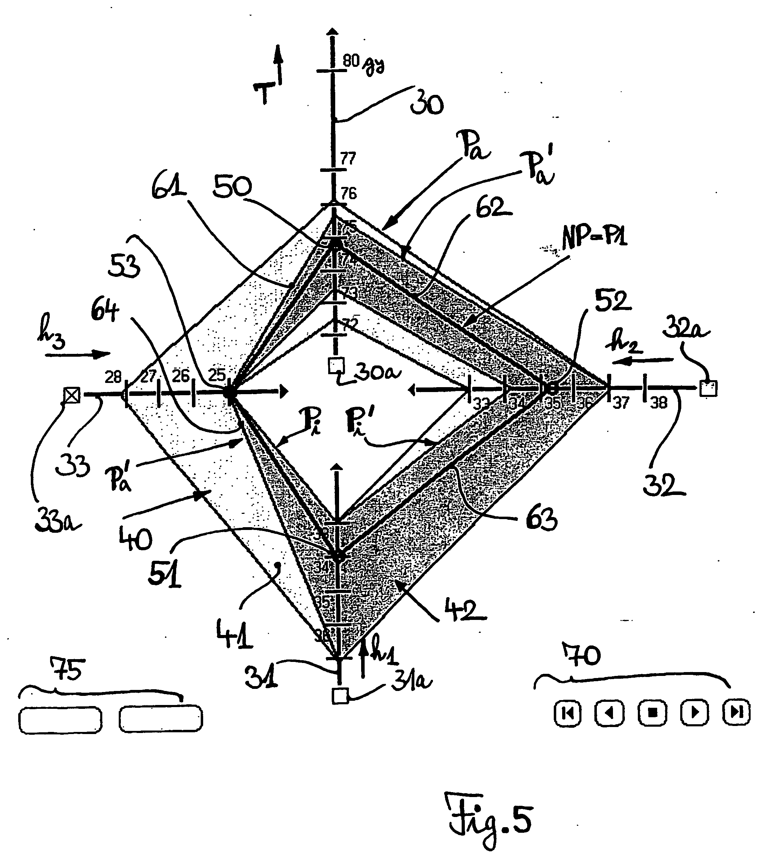

**Figur 4** ist eine schematische Repräsentation der Datenweitergabe und des Datenflusses, wie er aus Figur 1 im rechten Halbbild erkennbar ist. Eine Speicherbank oder eine Datenbank 1 entspricht derjenigen von Figur 1. Sie wird von einer Schnittstelle 22 über einen Datenpfad 23 beaufschlagt mit all denjenigen errechneten "Lösungen", die folgende Parameter umfassen:
Eine Anzahl von Bestrahlungsrichtungen im Sinne α₁ bis αₙ, wie in Figur 3a veranschaulicht.
Eine Vorgabe von Strahlendosen, die als Rastersegmente entsprechend den Figuren 2a bis Figur 2e für einen Kollimator oder einen Strahlungskopf 12 vorgegeben sind und dort einzustellen sind, sowohl hinsichtlich der Flächengestalt des Rasters, wie auch hinsichtlich einer jeweils zugehörigen Bestrahlungszeit, um Intensitätsverteilungen auch einstellen zu können, nicht nur Formen und Gestalt, entsprechend der Einstellung der Rasterblätter nach Figur 3. Werden pro Strahlungsrichtung αᵢ mehrere Datensätze übergeben, so entspricht das einer mehrfachen Überlagerung von unterschiedlichen Rasterbildem oder Flächenrastem, die zeitlich nacheinander an einem gleichen Winkel α überlagert werden und so in Summe gesehen einen in der Intensität verteilten Verlauf ergeben, wie er in den Figuren 2a bis 2e dargestellt ist. Das strukturelle Aussehen des Strahlenkopfes kann auch als "Strahlen-Pattern" pro Richtung angesehen werden, wobei jedes Raster des "Pattems" durch einen Grauwert so repräsentiert ist, daß eine bestimmte Strahlendosis zwischen einem Minimal- und einem Maximalwert hier an diesem Fleck oder Rasterelement erreicht wird.
Auch Daten über die Definition der Lage der Organe und ihrer räumlichen Erstreckung können von dem Daten-Interface 22 mit übergeben werden, z.B. separat von den eigentlichen Lösungen, zur visuellen Darstellung aus der Datenbank 1.

Ein Rechnersystem 20 gemäß Figur 4 berechnet die zuvor beschriebenen Steuergrößen zeitlich vorgelagert und verwendet dafür Eingangsgrößen D1 und CT. Die bei der Berechnung entstandenen Ergebnisse sind die "Lösungen" im zuvor beschriebenen Sinne, welche über die Datenleitung 21 an die zuvor beschriebene Einheit 22 weitergegeben werden.

Ausgangspunkte für die Vorab-Berechnung von Lösungen mit "Notenvektoren" im Sinne von Strahlendosen für die einzelnen Organe und Risiken sind Berechnungen pareto-optimaler Lösungen vom Rechnersystem 20, unter Vorgabe zumindest folgender Werte:
Vorgabe einer Startlösung E, bezogen auf Winkeleinstellungen α und Kollimator-Einstellungen des Kopfes 12, von der ausgehend eine Berechnung der mehreren pareto-optimalen Lösungen erfolgt, die über die Datenleitung 21 der Einheit 22 weitergeleitet werden.
Es werden Grenzwerte vorgegeben, die pro Organ definiert werden und sowohl Maximalwerte für Risikoorgane oder Minimalwerte für Zielorgane sind. Diese Grenzwerte können obere und untere Grenzwerte sein, können aber Minimalwerte ebenso sein, wie Maximalwerte. Diese Rahmenbedingungen werden mit D1 summarisch bezeichnet, die der Recheneinheit 20 zugeführt werden.
Ebenfalls zugeführt wird der Recheneinheit 20 eine Definition der Lage der Organe h₁,h₂,h₃ und des Zielvolumens T durch Vorgabe von CT-Diagrammen oder Schnitten, um ihre relative Lage und ihre räumliche Erstreckung festzulegen.
Es kann ebenfalls vorgegeben werden, wenn es nicht gesondert von der Einheit 20 berechnet wird, welche Winkellagen αᵢ als Strahlrichtungen verwendet werden sollen. Diese Größen fallen unter die Vorgabewerte D1.

Als Startlösungen E der Dimension p zu Beginn der Berechnung der pareto-optimalen Lösungen in der Rechnereinheit 20 kann beispielsweise ein schon bekannter Therapieplan für einen bestimmten Patienten mit einem bereits diagnostizierten Tumor Verwendung finden. Es kann auch eine empirische Lösung als Ausgangspunkt dienen, ebenso wie eine Standardlösung. Davon ausgehend werden unter Berücksichtigung der zuvor beschriebenen Randbedingung D1 der Dimension n und der physiologischen Parameter durch Computertomographie CT der Dimension m die pareto-optimale Lösungen berechnet, die über die Datenleitung 21 und die Einheit 22 nach Fertigstellung der Vorab-Berechnungen mit der Datenleitung 23 in die Datenbank 1 eingespeist werden, von der aus die visuelle Darstellung erfolgt, die im folgenden beschrieben werden soll.

Obwohl eine beliebige Anzahl von Zielvolumina verwendbar ist, soll die Beschreibung anhand von einem Zielvolumen T als Target erfolgen. Obwohl die Arbeitsweise auch mit (nur) einem Risikovolumen h₃ zusätzlich zum Zielvolumen T arbeitet, soll das Beispiel anhand von drei Risikovolumina im Sinne von drei Organen h₁,h₂ und h₃ erfolgen. Eine andere Umschreibung liegt so, daß zumindest zwei Volumen so zu bestrahlen sind, daß eines davon eine erheblich größere Strahlendosis erhält, als das andere. Sind mehrere Risikoorgane vorhanden, sind mehrere Risikovolumen mit einer so geringen Strahlenbelastung zu versehen und das eine Zielvolumen eine Strahlenbelastung erfährt, die wesentlich höher ist.

Beispiele liegen für eine Lunge bei etwa 33 Gray (gy), für Gewebe ohne Organstruktur etwas oberhalb und für riskantere Organe, wie Rückenmark bei 25gy und bei Sehnerven unter 10gy. Diese Grenzwerte gehen über die Parameter D1 in die Berechnung des Rechners 20 ein, der selbst mit Volumenelementen (Voxel) rechnet und aus der Belastung der einzelnen Voxel die Summenbelastung eines Organs oder - des Targets bestimmt, was mit einer Berechnung über finite (räumliche) Elemente verglichen werden kann.

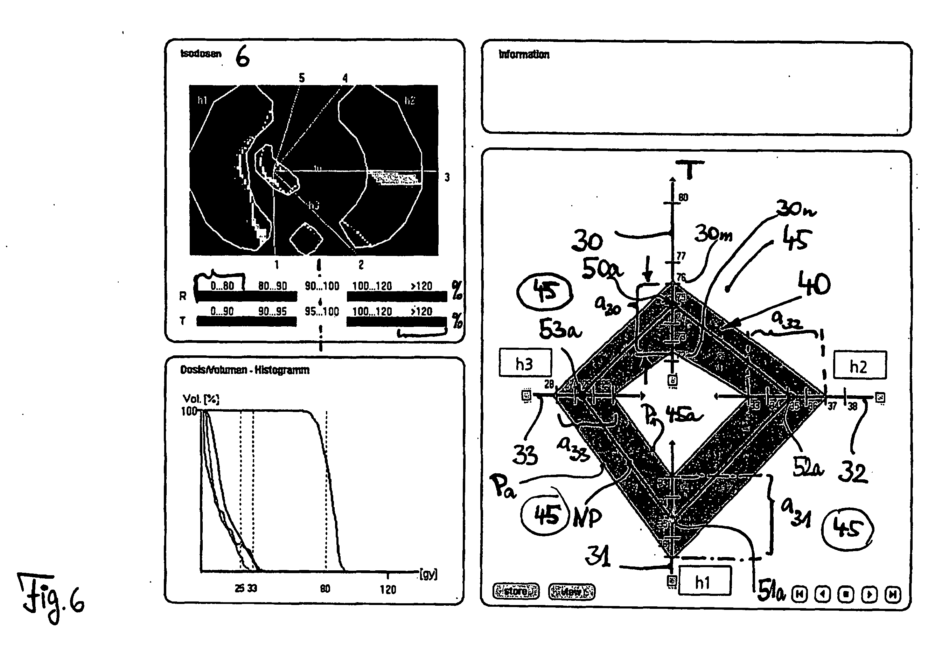

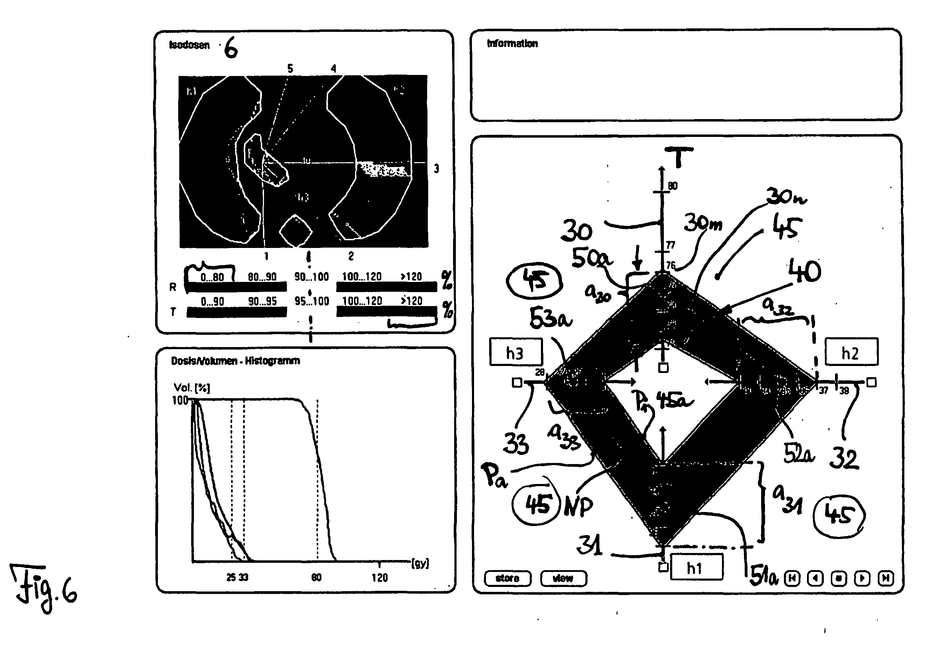

Figur 5 veranschaulicht eine Planungszone 40, die aus zwei Abschnitten 41 und 42 besteht Figur 5 ist bereits eine im weit fortgeschrittenen Stadium einer Planungssitzung erhaltene Darstellung an dem zuvor beschriebenen Schirm 3, die zunächst mit den von ihr verwendeten grafischen Elementen beschrieben werden soll, bevor auf eine Arbeitssitzung Bezug genommen wird, die mit der Figur 6 beginnen kann.

Dargestellt werden nicht die Steuergrößen, sondern eine "Lösung" eines Strahlentherapie-Plans, welcher selbst eine Vielzahl der besagten und zuvor eingehend erläuterten Steuergrößen und Steuerbefehlen für das Strahlentherapiegerät umfaßt. Diese in der Datenbank 1 vorgehaltenen Informationen werden nicht dargestellt, sondern es wird der Notenvektor in dem Sinne dargestellt, daß Kennwerte von Strahlendosen für ein Zielvolumen T und für mindestens ein Risikovolumen, hier die drei Risikovolumina h₁,h₂,h₃ zur Festlegung der Planungszone dienen. Die Planungszone 40 umschreibt mit den Kennwerten, die Strahlendosen entsprechen, bildlich die Vielzahl von technischen Steuergrößen, die hinter einer jeweiligen Lösung stehen, welche Lösung im hervorgehobenen Beispiel aus vier Strahlendosen 50,51, 52 und 53 besteht, die den drei Risikoorganen h₂,h₁,h₃ sowie dem Target T für diese Lösung zugeordnet sind.

Eine lineare Verbindung der vier beschriebenen Kennwerte ergibt im dargestellten Fall ein flächiges Polygon NP, das als Navigations-Linienkörper ein Viereck bildet, innerhalb der als Fläche hier dargestellten Planungszone, die mit einem innenliegenden und einem außenliegenden Polygon Pₐ und Pᵢ außen und innen begrenzt ist. Das Navigations-Polygon NP=P1 liegt innerhalb dieses Gebietes. Das Gebiet kann auch räumlich sein, es muß nicht zwingend Polygonstruktur haben, jedoch ist diese Struktur im beschriebenen Fall vorteilhaft hinsichtlich ihrer einfachen Überschaubarkeit, bei komplexer Vielfalt von dahinterstehenden Lösungen.

Im beschriebenen Beispiel wird das Navigations-Polygon NP durch die vier Punkte 50 bis 53 festgelegt. Jeder Punkt liegt auf einer der Achsen 30,31,32,33, wobei die Risikoachse 30 das Target T betrifft, zwischen Dosiswerten von etwa 72gy bis 80gy.

Die beiden Risikoachsen erstrecken sich über geringere Strahlendosen zwischen 25gy bis 28gy für h₃ und jeweils 33 bis 37gy für h₂ und h₁. Die aufgezeichneten Achsen liegen so, daß keine zwei Achsen identisch sind und daß benachbarte Achsen, beispielsweise die Achsen 32 und 31 oder die Achsen 30 und 33 nicht übereinanderfallen, um die Planungszone 40 zwischen dem inneren Polygon Pᵢ und dem äußeren Polygon Pₐ aufspannen zu können.

Innerhalb dieser Navigationszone ist beispielsweise die Verbindung vom Kennwert 50 für das Target auf der Achse 30 zum Kennwert 52 für das Risiko-Organ h₂ mit einer geraden Linie als Strecke 62 verbunden. Entsprechend erfolgt auch die Verbindung mit den anderen Punkten 51 und 53, jeweils zur benachbarten Achse und der dort angegebenen Kenngröße für die Strahlendosis der gerade dargestellten Lösung. So ist als Beispiel die Verbindungsstrecke 63 zwischen den Strahlendosen 52 und 51 auf den beiden Risikoachsen h₂ und h₁ repräsentiert. Die übrigen Verbindungsstrecken ergeben sich aus dem Zusammenhang und sind nicht gesondert beziffert.

Es ist für die Strahlenachsen, die jeweils eine Skala darstellen, nur ein Abschnitt gezeigt, der zumindest denjenigen Abschnitt enthält, in dem Kennwerte für Strahlendosen aus den gespeicherten Lösungen der Datenbank 1 vorhanden sind. Alle Lösungen zusammengenommen, oder zumindest einen wesentlichen Anteil der verfügbaren Lösungen, die zuvor gemäß Figur 4 von einem Rechner 20 erzeugt worden sind, bilden die Navigationszone 40 als einen im folgenden beschriebenen Navigationsraum so ab, daß die äußere Begrenzung Pₐ ist.

Es sollte dazu erwähnt sein, daß diese äußere Linie keine Lösung sein muß, sondern sich aus Punkten zusammensetzt, die von unterschiedlichen Lösungen stammen. Eine Lösung wird lediglich dadurch repräsentiert, daß ein inneres Navigations-Polygon NP, hier dargestellt als P1 für eine Lösung, visuell dargestellt wird. Die visuelle Darstellung erfolgt mit einem erkennbaren Kontrast gegenüber der Farbe oder dem Grauwert der Navigationszone. Diese wiederum ist farblich oder vom Grauwert her unterscheidbar oder erkennbar gegenüber dem Außenraum, der nicht in Frage kommt oder dem Innenraum, der ebenfalls nicht in Frage kommt, abgegrenzt.

Die beiden, als Umfeld zusammengefaßten Bereiche der visuellen Darstellung sind mit 45 und 45a in Figur 6 gekennzeichnet. Figur 6 zeigt dabei eine Vorstufe zu derjenigen von Figur 5, bei der noch keine Veränderung des Kennwertes 53a zum inneren Grenzwert 25 gy auf der Achse 33 erfolgte.

Hier ist ersichtlich, daß ein jeweiliges Intervall a₃₀,a₃₁ auf den Achsen 30 bzw. 31 gebildet worden ist, aus Kennwerten von Lösungen, die in der Datenbank 1 verfügbar sind. Entsprechendes gilt für die Achsen 32 und 33 und die Intervalle a₃₂,a₃₃. Diese als Akzeptanzintervalle bezeichneten Intervalle oder Abschnitte der Achsen 30 bis 31 bilden die Vorgaben für die als Fläche gezeichnete Navigationszone 40, die grau gegenüber dem Umfeld 45,45a dargestellt ist.

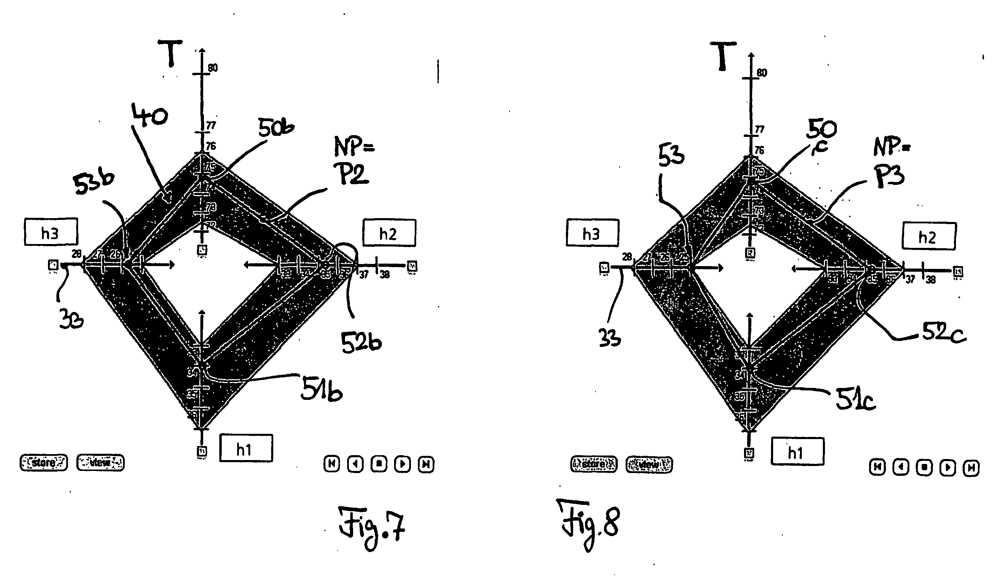

Das Navigations-Polygon NP ist hier als ein Polygon innerhalb der Zone eingezeichnet und ihr gegenüber hervorgehoben. Es wird nur eine Lösung dargestellt, die als Startlösung bezeichnet werden kann, von der ausgehend ein Benutzer eine Veränderung der dargestellten Lösung im Rahmen der folgenden Figuren 7ff vornimmt.

Dazu soll ausgegangen werden von gemäß Figur 6 eingestellten Kennwerten 53a auf der Skala h₃, 51a auf der Skala h₁ und 52a auf der Skala h₂, entsprechend den Achsen 33,31 und 32. Diese drei Risikoachsen sind im Winkel von 90° angeordnet. Demgegenüber ist senkrecht aufwärts eine Targetachse 30 gezeigt, deren Dosiswert als Kennwert 50a erkennbar ist.

Umschrieben in technischer Schreibweise heißt diese Lösung, daß dem Target eine Strahlendosis von 75gy für den Fall der Auswahl dieser Lösung zugewiesen würde, während die Risikoorgane die entsprechend aufgezeichneten Strahlendosen erhalten würden. Gewünscht wäre nunmehr ein oberer Randwert 30m auf der Targetachse, entsprechend 76gy und ein unterer (innerer) Randwert auf den übrigen Risikoachsen, was zusammen in einer Lösung aber nicht erreichbar ist, entsprechend den zuvor beschriebenen pareto-optimalen Lösungen der gespeicherten Menge von verfügbaren Lösungen. Auch herausgefallen ist dabei der gewünschte Strahlenwert 80gy auf der Targetachse, der ohne Verletzung der anderen Randbedingungen der Risikoachsen nicht in den vorausberechneten Lösungen erhalten werden konnte.

Erläutert werden soll noch der obere und untere Grenzwert 30m und 30n auf der Targetachse 30, wobei sich entsprechende obere und untere Grenzwerte auch auf den Risikoachsen 32,31 und 33 finden. Mit diesen Grenzwerten wird auf jeder Achse das Akzeptanzintervall eingegrenzt, so daß alle Kennwerte von Strahlendosen innerhalb oder zumindest auf der Randlinie der Planungszone 40 zu liegen kommt. Gesucht ist aus der Startdarstellung von Figur 6 eine neue Lösung, bei der - nach Wunsch des Benutzers und hier zur Veranschaulichung angenommen - die Strahlenbelastung für das Risikoorgan h₃ sinkt, also der Wert 53a zu kleineren Strahlenwerten hin verlagert werden soll. Gleichzeitig soll die Strahlendosis 50a für das Target erhöht werden. Beide Strahlendosen sollen mithin verbessert werden, eine soll reduziert werden und die andere erhöht werden.

Alle dazu verfügbaren und möglichen Lösungen sind in der Datenbank 1 vorhanden und dort vorab gespeichert. Sie werden im Verlaufe der folgenden Figuren 7ff jeweils individuell herausgegriffen, durch Verändern eines der Eckpunkte des Polygons NP von Figur 6.

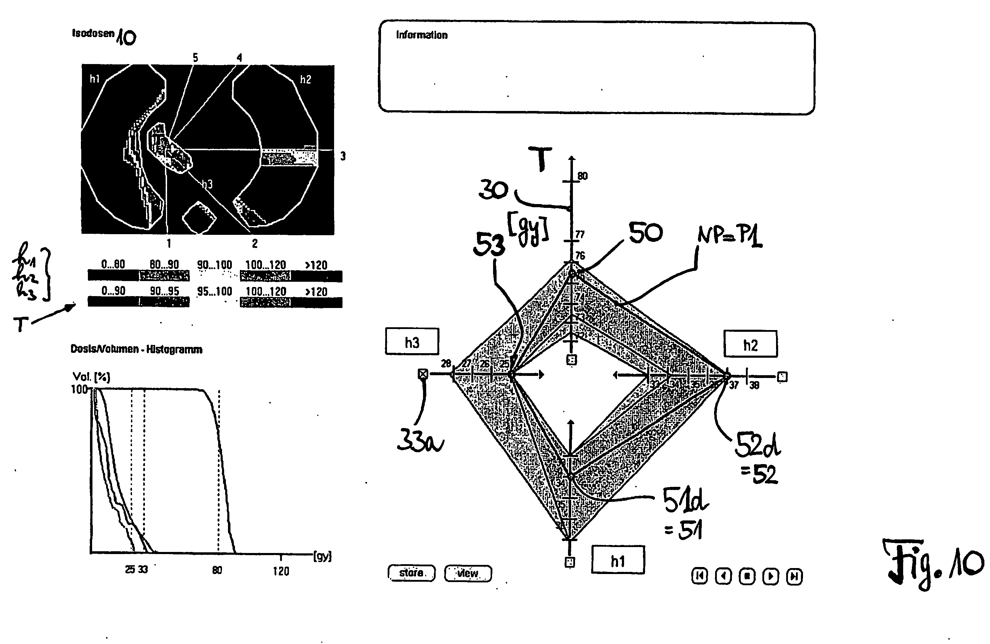

Bevor der Weg zum Verlassen der Figur 6 aber beschrieben wird, soll die Darstellung im linken Bereich der Figur 6 noch vertieft werden, die in Figur 2 bereits veranschaulicht wurde. Hinsichtlich der zweiten Darstellung auf der linken Seite (der unteren Darstellung) soll auf die Figur 11 verwiesen werden, die später erläutert wird. Bei dem sichtbar eingestellten Navigations-Polygon als ein möglicher Linienkörper, der eine Lösung der gespeicherten Lösungen der Datenbank 1 repräsentiert, ergibt sich eine Strahlenbelastung gemäß dem Transversal-Schnitt der linken oberen Ausschnittsfigur aus Figur 6. Diese entspricht der Figur 2. Es sind die fünf Richtungen α₁ bis α₅ erkennbar sowie die den Achsen 30 bis 33 entsprechende Volumina auf einer von vielen möglichen axialen Höhen. Es ergeben sich dabei auf dieser Transversal-Ebene die lokalen Strahlenbelastungen, die mit h₂₂, h₂₁, h₁₁ in Figur 2 durch unterschiedliche Grauwerte repräsentiert sind, wobei eine Skala am unteren Rand anzeigt, ob für das Risikoorgan in Voxelzonen Abweichungen nach oben oder nach unten und für das Target Abweichungen nach oben oder unten erreicht werden, wenn die im Polygon dargestellte Lösung ausgewählt und für eine spätere Therapie verwendet werden würde.

Diese unterschiedlichen Strahlendosen auf der Transversal-Ebene können farblich gekennzeichnet werden, wobei Abweichungen nach unten bei Risiko R und Abweichungen nach oben bei Target T dieselben Farben erhalten können. Dadurch wird die jeweilige Zielrichtung mit derselben Farbe dargestellt, während die anderweitigen Abweichungen mit einer anderen, aber ebenfalls derselben Farbe gekennzeichnet werden können. Auf die Farbdarstellung der Figur 6 wird zur Erläuterung verwiesen. Ein Blick auf den Transversal-Schnitt zeigt dem Beobachter die physische Wirkung des eingestellten Polygons NP, das als solches abstrakt und losgelöst von der Physik, aber leicht zu handhaben ist. Eine Veränderung der axialen Höhe in Richtung 100 der Figur 1 entspricht einem anderen Transversal-Schnitt und würde eine andere Verteilung der Strahlendosen ergeben, bei unverändertem Navigations-Polygon NP.

Auf der einen beigefügten farbigen Figur 6 sind die Zuordnungen von 0 bis 80% hinsichtlich der Risiken h₁,h₂ und h₃ mit derselben Farbe gekennzeichnet, wie das Target oberhalb 120%. Im Target T ist nach der bildlichen Darstellung der überwiegende Anteil zwischen 95% und 120%, während der überwiegende Volumenanteil bei den Risiken im Bereich unter 80% liegt. Die dargestellte farbliche Kennzeichnung des zu verbessernden Strahlenwertes für das Risiko h₃ entspricht einer Verlagerung des Punktes 53a nach innen, in Richtung zu dem dort dargestellten (grünen) Pfeil, der eine Verbesserung anzeigt. Entsprechende Pfeile können auch in den anderen Risikoachsen vorgesehen sein, während ein gegensätzlicher Pfeil auf der Targetachse T die Zielrichtung für eine dortige Verbesserung andeutet.

Die Planungssitzung und das Planungswerkzeug arbeiten bei der Verbesserung von Figur 6 in Richtung einer Verringerung der Dosis von h₃ und einer Erhöhung der Dosis von T so, daß beispielsweise der Eckpunkt 53a vom Benutzer verlagerbar ist, also so auf der bildlichen Darstellung erkennbar und sensitiv ist, daß mit einem Steuerwerkzeug, wie der Maus oder einem Pad 4 diese Stelle ergriffen und in die Wunschrichtung verschiebbar ist, so daß sie beispielsweise bei 53b unter einem Strahlenwert von 25,5 gy zu liegen kommt, bei unveränderter Planungszone 40 und auch unveränderter Skalierung der Achsen 30 bis 33.

Das entspricht einer Richtung zu einer anderen darzustellenden Lösung, die aber nicht zwingend einen Eckpunkt auf demjenigen Punkt 53b besitzen muß, wie vom Benutzter verlagert. Das Planungswerkzeug, bzw. die hinter ihr stehende Hardware oder Software in der Steuerungseinrichtung 5 läßt dazu einen Punkt zu, der demjenigen Punkt am nächsten ist, zu dem der Benutzer den Punkt 53a auf der Achse 33 verschoben hat. Dazu bietet sich für eine Datenbank eine Sortierung nach dem h₃-Wert an und die Auswahl des nächstliegenden Punktes. Aufgrund der vorgegebenen Richtung entspricht das einer Verengung des Anfangs eines neu zu bildenden Navigations-Polygons, das hier als P2 in Figur 7 schon entstanden dargestellt ist. Die dargestellte Lösung aus den Punkten 53b, 50b, 52b und 51 b, entsprechend dem Notenvektor mit den Kennwerten für die Strahlungsdosen für die einzelnen Achsen, entspricht einer vorgespeicherten Lösung, die hinsichtlich des Wertes 53b am ähnlichsten zu der vom Benutzter verlangten neuen Lösung ist.

Die anderen Punkte 50b,52b und 51 b werden nach einer Suchstruktur so ermittelt, daß alle diese drei übrigen Punkte möglichst nahe an den zuvor geltenden Strahlenwerten der gleichen Achsen, hier die Werte 50a,52a und 51 a von Figur 6 liegen, aber alle gemeinsam zu einer Lösung gehören, die den Strahlenwert 53b besitzt (oder ihm am nächsten kommt). Die Suchsteuerung ist damit in der Lage, das Navigations-Polygon sanft so zu verändern, daß es auf derjenigen Achse, auf welcher der Benutzer verändert, große Veränderung erfahren kann, während es auf den übrigen Achsen nur diejenigen Änderungen erfährt, die hinsichtlich der durch den Punkt 53b vorgegebenen Lösungsvielfalt noch verfügbar ist. Entsprechende Datenbank-Suchstrukturen erlauben - durch Sortierkriterien und Auswahl - das Berechnen einer Minimal-Abweichung der Summe der Einzelabweichungen an den Punkten 51a,52a und 50a gegenüber der dargestellten Lösung und damit eine Auswahl einer einzigen Lösung als neues darzustellendes Navigations-Polygon P2. Andere Suchstrukturen und Änderungen für die übrigen Punkte, die nicht aktiv verlagert worden sind, sind ebenfalls möglich, hier ist nur eine von mehreren Möglichkeiten näher erläutert worden.

Es ist auch möglich, einen der anderen Kennwerte 50a,52a und 51a aus Figur 6 zu verändern, so daß sich dann die entsprechenden jeweiligen übrigen drei Werte verlagern, unter Beachtung der Darstellung nur einer Lösung, die der zuvor Dargestellten zumindest ähnlich ist.

Hinsichtlich der Folgeänderung in den Nebendarstellungen zu den Isodosen und zu dem Dosis/Volumen-Histogramm wird eine entsprechende Darstellung auch zu Figur 7 erfolgen, so daß optisch nachvollziehbar ist, welche Strahlenbelastungen in welchen Transversal-Schnitten sich verändern, durch Verändern der Lagen, Bereiche und Größe der einzelnen Farbzuordnungen, wie anhand Figur 6 erläutert.

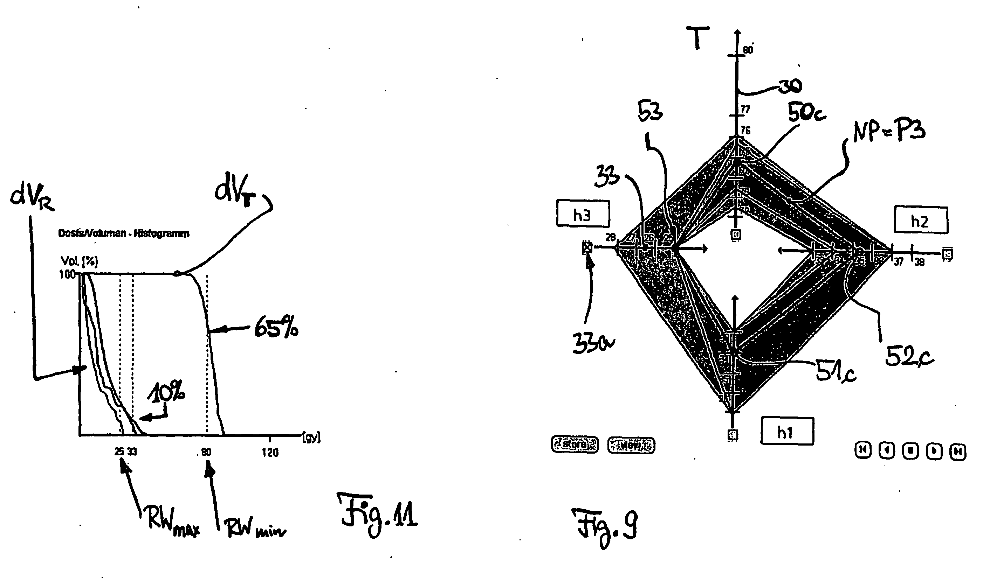

Es ist dann aus Figur 7 weiterhin erwünscht, die Dosis auf der Achse 33 zu h3 weiter zu verbessern, also zu reduzieren, in Richtung des dargestellten Pfeils. Bei dieser Veränderung hin zur **Figur 8** soll davon ausgegangen werden, daß der Benutzer den Punkt 53b an das innere Ende zur Mindestdosis 25gy hin verlagert und dort das Steuerwerkzeug 4 so beeinflußt (z.B. den Mausklick löst), daß dieser Punkt 53b hier als 53 zu liegen kommt. Eine entsprechende Veränderung des Navigations-Polygons NP hin zu einer neuen Geometrie P3 mit den Eckpunkten 53,50c,52c und 51 c ist unmittelbar aus der Figur erfaßbar. Aufgrund der Suche nach möglichst ähnlichen Werten auf den anderen Achsen 30,31 und 32 bleiben die dort dargestellten Kennwerte als Dosiswerte praktisch unverändert, obwohl sie Bestandteil einer andern Lösung sind, die den Kennwert 53 einschließt, der zuvor von dem Polygon P2 nicht umfaßt war. Es ist also in der Lösungsvielfalt der Datenbank 1 eine Lösung vorhanden gewesen, die eine minimale Dosisbelastung von 25gy auf der Rückenmark-Skala h₃ (Achse 33) ermöglicht, bei ebenfalls vernünftigen Belastungen der anderen Risiken h₁ und h₂, bei einer allerdings noch zu geringen Strahlenlast für das Target T, mit näherungsweise 75gy.

Der Übergang von der Figur 7 zur **Figur 8** soll hinsichtlich einer grafisch schlecht darstellbaren weiteren Funktionalität mit Worten beschrieben werden. Während bei dem Übergang von Figur 6 zur Figur 7 eine im wesentlichen schlagartige Veränderung des Polygons von Figur 6 zum neuen Navigations-Polygon P2 angenommen war, ist die Veränderung des Polygons von P2 nach P3 mit einer alternativen Darstellungsweise versehen, die auch für sämtliche Änderungen der Polygone Anwendung finden kann, ebenso wie die schlagartige Veränderung auch für alle Einzelschritte möglich ist.

Eine stetige oder kontinuierliche Veränderung des einen Polygons zum nächsten Polygon arbeitet so, daß der Benutzer visuell erfährt oder beobachten kann, wie sich ein Polygon P2 z.B. ausgehend vom "verzogenen" Punkt 53b zum Punkt 53 hin insgesamt verändert. Die beiden vom Punkt 53 ausgehenden Linien beginnen vom Punkt 53 aus vom alten Polygon P2 auf die Linien des neuen Polygons überzuwechseln, was einem Wisch-Effekt oder einem stetigen Übergangseffekt gleichkommt, der so langsam ablaufen soll, daß man ihn sichtbar mitbekommt. Die erste stationäre Darstellung von Figur 7 verändert sich dabei in einer Übergangsphase auf die zweite stationäre Darstellung von Figur 8.

Alle sichtbaren Linien des Polygons P2 innerhalb der Planungszone 40 werden dabei von links übergeblendet in alle Linien des Polygons P3. Die Zeit der Überblendung ist eine Übergangsphase, bei der nicht nur eine Lösung aus der Datenbank, sondern zwei Abschnitte von zwei Lösungen aus der Datenbank gleichzeitig, aber jeweils nicht vollständig, sondern in ihren Anteilen stetig verändert dargestellt werden.

Ein Verziehen von Punkt 53b in die andere Richtung (zu 53a) läßt den Wischeffekt umgekehrt beginnen, bei Punkt 52b (von rechts).

Die technische Funktion des Überblendens sorgt für eine leichtere grafische Erfaßbarkeit durch den Benutzer und erweckt ein Gefühl der Richtung und der Qualität des Wechsels, ohne erneut zum bisherigen alten Punkt zurückwechseln zu müssen, um den Unterschied durch ein Hin- und Herbewegen der Punkte 53 und 53b zu erfassen.

Aus der Figur 8 soll eine weitere Verbesserung jetzt erwünscht sein, die eine Erhöhung der Dosis auf der Target-Skala sein soll, wie an **Figur 9** veranschaulicht. Hier sind zunächst unveränderte Eckpunkte des Polygons P3 eingezeichnet. Sie sind unverändert, weil vor einem weiteren Verändern anderer Eckpunkte als desjenigen auf der Achse 33 ein Blockieren von Rückwärtsschritten auf der gerade optimierten h₃-Achse gesperrt werden soll.

Diese Sperrung wird durch ein Anklicken des Abschnitts 33a erreicht, der der Achse 33 zugeordnet ist. Entsprechende Bereiche finden sich nach der Darstellung von Figur 5 auch an den anderen Achsen, namentlich die Bereiche 31 a,32a und 30a, jeweils dem Ende der Skala zugeordnet, die eine Verschlechterung anzeigt, um mit den symbolisch dargestellten Pfeilen am jeweils anderen Ende die Zielrichtung der Verbesserung kenntlich zu machen.

Ein Anklicken des sensitiven Bildbereiches 33a sperrt schlechtere Werte als diejenigen, die mit der Einstellung 53 als Kennwert für eine zugehörige Strahlendosis schon optimiert worden sind. Dadurch ergibt sich eine Veränderung der Planungszone 40, weil alle Lösungen jetzt ausgenommen werden, die solche Dosiswerte auf der Skala 33 besitzen, die zwischen dem Punkt 53 und dem maximal möglichen Punkt des oberen Endes des Aktzeptanzintervalles a₃₃ von Figur 6 liegen. Diese Werte sind nach dem Aktivieren der Sperrfunktion auf der Darstellung nach Figur 9 mit einer anderen Farbe gekennzeichnet, hier leicht grau hinterlegt, während die übrige (die verbleibende) Planungszone noch dieselbe Farbe gegenüber der Umgebung besitzt.

Deutlicher dargestellt ist das an Figur 5, wo sich ein erster ausgeblendeter Abschnitt 41 oberhalb der beiden Verbindungsstrecken 64 und 61 ergibt, die zu den benachbarten Achsen von dem festgelegten Punkt 53 reichen. Der ausgeblendete Flächenbereich 41 erstreckt sich bis zum Rand an das Polygon Pₐ und erfaßt aufgrund der gesperrten Kennwerte in diesem Bereich auch Abschnitte in den übrigen Zonen des Polygons 40, namentlich auf derjenigen Seite, die den Hauptanteil der verbleibenden Planungszone 42 besitzt. Das hat seine Ursache darin, daß Lösungen mit Dosiswerten auf der Achse 33 sich mit anderen Dosiswerten auf den übrigen Achsen 30,32 und 31 auch in solche Bereiche erstrecken, die dort jetzt nicht mehr erhalten werden können. Eine Gesamtlösung mit vier zusammengehörigen Dosis-Kennwerten wird bereits dann gesperrt, wenn sie einen Punkt als Kennwert besitzt, der betragsmäßig oberhalb des Punktes 53 auf der Achse 33 liegt.

Es ergibt sich demnach eine reduzierte Planungszone 42, die innerhalb eines neuen äußeren Umrisses Pₐ' zu liegen kommt, wie in Figur 5 größer dargestellt und in Figur 9 durch eine unterschiedliche Grauwert-Belegung gekennzeichnet. Es ergibt sich auch eine neuere innere Randlinie Pᵢ', die sich hauptsächlich auf der rechten Seite der Figur 5 befindet, während die neue äußere Grenzlinie Pₐ' hauptsächlich auf der linken Fläche zu liegen kommt. Das ist aber abhängig davon, welcher der verlagerbaren Kennwerte 50 bis 53 für eine Fixierung oder Haltefunktion ausgewählt wird. Die entsprechende Zuordnung ist leicht aus der zuvor gegebenen Beschreibung und einer möglichen Drehung des Diagramms nach Figur 5 nach rechts oder links ersichtlich.

Es versteht sich, daß der gesperrte Bereich 41 auch wieder aktiviert werden kann, wenn die Lock-Funktion durch Anklicken des Bildabschnitts 33a der Figur 9 nochmals aktiviert wird, im Sinne einer Toggle-Funktion, so daß sich wieder ein Bild nach Figur 8 ergibt. Es können auch mehrere Punkte in dem Polygon festgelegt werden, bezogen auf die Skalenwerte 50c, 52c und 51 c von Figur 9, die dann weitere Abschnitte aus der Planungszone herausnehmen, um eine nochmals reduzierte Planungszone zu erreichen.

Für die folgende Beschreibung soll von einer jetzt aus Figur 9 heraus abgeleiteten weiteren Verbesserung der T-Achse 30 ausgegangen werden. Die Dosis für das Target ist nach der Kennwert-Darstellung 50c noch zu gering. Es wird deshalb eine Verlagerung in Richtung des dieser Achse zugeordneten Pfeils so vorgenommen, daß die Figur 10 entsteht, bei der der Punkt 50c auf den Punkt 50 gelegt wird, bei Beibehalten der Lock-Funktion der Achse h₃, also eines Festlegens des Punktes 53.

Mit oder ohne einen fließenden Übergang wird die Figur 10 dann gebildet, die eine neue Punktegeometrie 53,50, wie beschrieben, und die Punkte 51d und 52d, entsprechend den Punkten 51 und 52 von Figur 5 besitzt. Dadurch ergibt sich das Polygon P1 als Navigationskörper, wie schon in der Figur 5 dargestellt. Diese gefundene Lösung, dargestellt durch das Polygon P1 entspricht einem gefundenen Optimum, bei dem zwar gegenüber der Figur 9 die h₂-Achse deutlich verschlechtert wurde, aber die h1-Achse unverändert blieb, die h3-Achse minimiert wurde und die Targetachse 30 auf das bestmögliche Optimum 50 (entsprechend einem Wert von 75gy) optimiert werden konnte.

Die dieser Einstellung, also der gefundenen Lösung entsprechenden Steuerwerte für einen Strahlen-Therapieplan, der später zum Einsatz kommen kann, nachdem er über die Steuerleitungen 6,7 in das Therapiegerät übertragen wurde, entspricht dabei einer Auswahl von Steuergrößen für die Einstellung des Strahlenkopfes an den unterschiedlichen Gerätewinkeln αᵢ und entspricht damit einer vorgegebenen Intensitätsverteilung, wie es aus Figur 3a mit drei unterschiedlichen Richtungen schematisch erkennbar ist, aber für das hier beschriebene Beispiel anhand der Figur 2 aus fünf unterschiedlichen Richtungen Strahlungseinflüsse beinhaltet, verbunden mit entsprechender Intensitätseinstellung gemäß den Figuren 2a bis 2e.

Die beschriebenen Figuren 6 ff enthalten so, wie in Figur 5 erläutert, auch Archivierungsfunktionen 75 und Monitoring-Funktionen 70, die entsprechend einem Aufzeichnungsbefehl, einem Wiedergabebefehl oder einem Ablaufen von zuvor aufgezeichnetem Navigationspolygonen NP entsprechen. Dazu werden bei einem entsprechenden "Klick" auf die Sichtbereiche am Bildschirm über das Handhabungsinstrument und den entsprechenden Mauszeiger Pointer auf bestimmte Lösungen zwischengespeichert. Die Abspeicherung aktueller Navigations-Polygone, die für eine spätere Neubetrachtung noch einmal aufgehoben werden sollen, geschieht mit dem Store-Bereich auf dem Sichtschirm 3. Ist zumindest ein Navigations-Poylgon NP über den Store-Bereich gespeichert, kann durch ein Anklicken des Abschnitts "View" des Funktionsbereiches 75 in ein Wechseln in das Anzeigen gespeicherter Funktionen erhalten werden. Das Speichern bzw. Referenzieren geschieht durch die beschriebenen Pointer (Zeiger) auf den Datenbanksatz, der eine der in Speicher 1 gespeicherten Lösungen ist. Diese Speicherung kann in einer ersten Log-Datei erfolgen und bei Anklicken eines View-Bereichs am Schirm 3 wechselt das Planungswerkzeug, also die Bildschirmdarstellung auf dem Bildschirm 3, in dem View-Modus und zeigt die jeweiligen Navigations-Polygone NP an, die zuvor über den Store-Bereich ausgewählt wurden. Die entsprechende Gestalt der zugehörigen Planungszone 40 oder nur 42 (abzüglich des Abschnitts 41) wird zugehörig dargestellt.

Durch den Recorder-Button im Abschnitt 70 der Bildschirmdarstellung kann ein Vorwärts oder ein Rückwärts innerhalb der Log-Datei erfolgen. Es können so neue Anfangspunkte für neue Optimierungssuchen gewählt werden, die einer früher schon einmal gefunden Lösung entsprechen, die zwischengespeichert wurde, bevor man in eine seinerzeit als zuversichtlich vermutete Richtung weiter optimieren wollte, was dann zu keinem sinnvollen Ergebnis geführt hat. Eine Rückkehr zu dem zuvor gespeicherten Ausgangspunkt als NP ist dabei hilfreich.

Neben der einen beschriebenen Log-Datei kann auch eine sequentielle Log-Datei automatisch mitgeführt werden, die jeden Entwicklungsschritt des NP in der Planungszone verfolgt und aufzeichnet, so daß man durch Vorwärts-, Rückwärts- und Anfangs- und Ende-Schaltbereiche im Funktionsabschnitt 70 eine Steuerung erreichen kann. Das Abrufen früher einmal gesehener Lösungen wird erleichtert.

Obwohl zuvor ein Schwerpunkt auf den in Figur 5 dargestellten Abschnitt einer Bildschirmdarstellung gelegt worden ist, soll die zugehörig auch dargestellte Nebeninformation nicht vernachlässigt werden. Dies zeigt die aus dem sehr abstrakten Denkmodell der Planungszone näher an die Realität gerückte Isodosen-Darstellung und Dosis-Volumen-Darstellung der beiden links in Figur 6 sichtbaren Diagramme. Die jeweils in der globalen Planungszone 40 markierte Polygon-Darstellung NP ist Ausgangspunkt und schafft eine leichte optische Visualisierung der in der Datenbank auch noch zur Verfügung stehenden und dem Benutzer vertrauteren Visualisierung von als solchen bekannten Isodosen und Volumen-Histogrammen.

Ein physikalisches Therapie-Setup ist im Hintergrund auch gespeichert und kann ggf. in einem physikalischen Planungsfenster eingesehen werden, unter "Information".

Während die Darstellung nach Figur 5 zweidimensional bevorzugt arbeitet, ist bei den Isodosen die zweidimensionale Darstellung durch unterschiedlich liegende Tansversal-Schnitte in die dritte Dimension erweiterbar. Ebenso ist die DosisNolumen-Histogrammdarstellung gemäß Figur 11 dazu in der Lage, die flächige Darstellung von Figur 5 dem Benutzer anschaulich in die dritte Dimension zu transferieren, wobei die Dosis auf der Abszisse und der zugehörige Volumenanteil auf der Ordinate aufgetragen ist. Beispielsweise ist die Dosis/Voiumen-Verteilung als Graph dV_{T} so gestaltet, daß die erwünschten 80gy in 65% des Volumens des Targets T erhalten werden, während 35% mit einer geringeren Dosis beaufschlagt werden. Die vorgegebenen Grenzwerte RWₘₐₓ für die Risiken und RWₘᵢₙ für das Target sind auf der horizontalen Achse aufgetragen und daraus ist ersichtlich, daß für die Organe (die Risikovolumina) ein Verlauf von dV_{R} so erhalten wird, daß weit weniger als 20% bis hin zu 10% über dem Maximalwert der Strahlenbelastung liegt.

Aufgrund der Darstellung als Volumen ist die dritte Dimension in dieser Graphik enthalten. Eine einer Vielzahl von möglichen zweidimensionalen Schnitten ist die Isodosen-Darstellung. Eine reine zweidimensionale Darstellung in der Planungszone 40 ist der Ausgangspunkt für die zuvor beschriebenen zwei Hilfsdarstellungen.

## Patentansprüche

1. Verfahren zur Steuerung über eine visuell wahrnehmbare Darstellung und zur interaktiven Auswahl von Steuergrößen (x₁,y₇,α₁ ,α₂,α₃, I₁,I₂) eines Strahlentherapieplans aus einer Datenbank (1) mit einer Vielzahl von vorberechneten oder vorgegebenen Lösungen,
wobei jede Lösung
(i) einen Strahlentherapieplan repräsentiert, der in technischer Hinsicht selbst aus einer Vielzahl von Steuergrößen oder Befehlen besteht, die einem Strahlentherapiegerät (10,11,12) beaufschlagbar sind (6,7) oder für dieses Gerät (10) zur Verfügung gestellt werden, zeitlich vor einer eventuellen Ausführung der Steuergrößen oder Befehle im Rahmen einer Durchführung einer Therapie;
(ii) Kennwerte von Strahlendosen für ein Zielvolumen oder Target (T) und mindestens ein Risikovolumen (h₁,h₂,h₃) enthält, die in der Datenbank (1) gespeichert sind;
und wobei
(a) mehrere Achsen als Strahlendosisskalen (30,31,32,33) für das Zielvolumen (T) und das zumindest ein Risikovolumen (h₁,h₂,h₃) auf einer Darstellungseinrichtung (3; 3a,3b) sichtbar aufgetragen oder dargestellt werden, zur Bildung von zumindest einer Risikoachse und Targetachse, wobei keine Achse mit einer anderen Achse übereinanderfällt und benachbarte Achsen (30,32;30,33) nicht parallel verlaufen;
(b) die Kennwerte (50,51,52,53) der Strahlendosen für zumindest eine Vielzahl der gespeicherten Lösungen so den jeweils zugehörigen Achsen zugewiesen werden, dass für jede Risikoachse (31,32,33) und die Targetachse (30) ein Akzeptanzintervall (a₃₀,a₃₁,a₃₂,a₃₃) entsteht, die für alle Achsen gemeinsam eine Planungszone (40;41,42) festlegen;
(c) die Planungszone (40) auf der Darstellungseinrichtung (3) gegenüber dem Umfeld (45,45a) hervorgehoben oder gegenüber dem Umfeld unterscheidungsfähig sichtbar dargestellt wird.

2. Verfahren nach Anspruch 1, wobei jede Achse (30 - 33) einen Abschnitt als Akzeptanzintervall aufvveist, der innerhalb der Planungszone (40) liegt und jeder Abschnitt einen oberen und einen unteren Randwert (30m,30n) aufweist.

3. Verfahren nach Anspruch 1 oder 2, wobei die Planungszone als eine Planungsfläche eine polygonförmige Gestalt aufweist, mit innerem und äußerem Randpolygon (Pᵢ,Pₐ).

4. Verfahren nach Anspruch 1, wobei Verbindungsstrecken von Intervallenden oder der Randwerte nach Anspruch 2 von benachbarten Achsen (30,32) eine äußere und eine innere Begrenzung der Planungszone festlegen, zur erfassbaren Visualisierung der in der Datenbank gespeicherten Lösungsvielfalt.

5. Verfahren nach Anspruch 1, wobei innerhalb der Planungszone im Zustand einer **ersten stationären Darstellung** nur eine Lösung aus der Datenbank so dargestellt wird, dass ihre Kennwerte (50,51,52,53;50b - 53b) von Strahlendosen auf den Achsen (30 - 33) mit sichtbaren Linien verbunden werden, zur Bildung eines Navigations-Linienkörpers (NP,P1,P2) als ein Polygon.

6. Verfahren nach Anspruch 5, wobei alle Verbindungslinien innerhalb der Planungszone (40) liegen.

7. Verfahren nach Anspruch 5 oder 6, wobei die Verbindungslinien geradlinig verlaufen, um das Polygon (P1) als Navigations-Linienkörper mit einer gleichen Eckenzahl zu bilden, wie eine Anzahl von äußeren Ecken (Pa, Pa') der Planungszone (40).

8. Verfahren nach Anspruch 5, wobei in einem Übergangszustand von der ersten stationären Darstellung zu einer zweiten stationären Darstellung (50c - 53c) eine Form oder Lage des ersten Linienkörpers (NP) verändert und ein zweiter Linienkörper gebildet wird (P3), aber innerhalb der Planungszone (40) verbleibt, zur Darstellung einer zweiten Lösung aus der Datenbank mit ihren zugehörigen Kennwerten für Strahlendosen von Zielvolumen und Risikovolumen.

9. Verfahren nach Anspruch 8, wobei während des Übergangszustandes ein Wechsel von dem ersten Linienkörper (P2) zu dem zweiten Linienkörper (P3) fließend oder allmählich erfolgt, um den Wechsel anschaulich und sichtbar zu machen, und im sichtbar fließenden Wechsel zwei Lösungen oder zugehörige Linienkörper (P3,P2) jeweils zumindest abschnittsweise gleichzeitig sichtbar sind.

10. Verfahren nach Anspruch 1, wobei jede Lösung für eine Anzahl von Dosisverteitungen für mehrere Winkellagen (α₁ - α₅) steht, zur nicht unmittelbaren Weitergabe an einen Strahlenkopf (12) eines Therapiegerätes (10,11), insbesondere an zugehörige Mehrfachstreifen (13,14) eines Multileaf-Kollimators vor einem Strahlenkopf (12).

11. Verfahren nach Anspruch 1, wobei in der Planungszone (40), die vom Hintergrund (45a,45) insbesondere farbig oder vom Grauwert her abgesetzt ist, während eines Zustandes einer stationären Darstellung nur eine einzige der Vielzahl von gespeicherten Lösungen dargestellt wird.

12. Verfahren nach Anspruch 1, wobei ein erster Abschnitt (41) der Planungszone gesperrt wird oder sperrbar ist, um die verbliebene Planungszone (42) für Veränderungen eines in der Planungszone (40) einbeschriebenen Navigationskörpers (NP) zu belassen.

13. Verfahren nach Anspruch 12, wobei die Sperrung wieder aufhebbar ist, um den ersten Abschnitt (41) dem verbliebenen Abschnitt für Veränderungen des einbeschriebenen Navigationskörpers wieder hinzuzufügen.

14. Verfahren nach Anspruch 12 oder 13, wobei die Sperrung des ersten Abschnitts durch Blockieren eines Kennwertes (53) auf einer der Achsen (33) erfolgt, wodurch der Kennwert (53) zu einem Scheitelpunkt wird und eine neue Ecke eines neuen äußeren Polygons (Pₐ') bildet und bestimmte Lösungen der Datenbank gesperrt werden
(i) für eine Darstellung als Navigations- Linienkörper (NP) und
(ii) für eine Auswahl,
wobei die bestimmten Lösungen einen Kennwert als Dosis-Kennwert auf der einen Achse (33) besitzen, der im gesperrten Abschnitt (41) liegt.

15. Verfahren nach Anspruch 1, wobei die Kennwerte (50,51,52,53) der Strahlendosen durch EUD-Werte als gemittelte Dosisverteilungen pro Organ oder Target repräsentiert sind.

16. Verfahren nach Anspruch 5, wobei durch ein Betätigen eines dargestellten begrenzten Steuerabschnitts (75) die Speicherung einer Nummer der aktuell dargestellten Lösung im aktuellen Navigations-Linienkörpers (P1) initiiert wird.

17. Verfahren nach Anspruch 16, wobei durch Betätigen eines anderen begrenzten Steuerabschnitts (75) der Darstellung in einen Sicht-Modus umgeschaltet wird, um von der Datenbank (1) die Lösungen zu den gespeicherten Nummern zur Darstellung (3,3a) bereitzustellen, die unter Verwendung des ersten Steuerabschnitts des Sichtschirms (3) gespeichert wurden.

18. Verfahren nach Anspruch 17, wobei mit Hilfe von Recorder-Abschnitten (70) auf einem Sichtschirm als Darstellungseinrichtung die den zugehörigen gespeicherten Lösungen entsprechenden Visualisierungen nach einem Anwenderwunsch dargestellt werden.

19. Verfahren nach Anspruch 5, wobei Lösungen, insbesondere Zeiger auf gespeicherte Datensätze der Datenbank, welche Lösungen einem aktuell dargestellten Navigations-Linienkörper (P2) zugeordnet sind, in einem Hintergrund automatisch in einer Log-Datei sequentiell gespeichert werden (5,1), bei einem Betätigen eines Steuerabschnitts.

20. Verfahren nach Anspruch 19, wobei das Betätigen eines von mehreren Recorder-Bildabschnitten (70), deren Funktionen Recorder-Funktionen entsprechen, die Visualisierungen der in der Log-Datei referenzierten Lösungen nach Anwenderwünschen abgerufen werden.

21. Verfahren nach Anspruch 20, wobei das Betätigen eines vorher bereits betätigten Steuerabschnitts eine Umschaltung in einen Modus veranlasst, der eine Fortsetzung der Navigation in der aktuellen Planungszone gestattet.

22. Verfahren nach einem der Ansprüche 19 bis 21, bei dem eine Betätigung ein Anklicken mit einem Mauszeiger oder ein Berühren des Sichtschirms an einer spezifischen Stelle als Sichtschirms (3) als Darstellungseinrichtung ist.

23. Verfahren nach Anspruch 12, wobei die Sperrung eines ersten Abschnitts (41) durch ein Sperren eines schlechteren Skalenwertes erfolgt, bezogen auf Dosis und Dosisrichtung auf einer jeweiligen Achse (30 - 33).

24. Verfahren nach Anspruch 5, wobei in einer zweiten stationären Darstellung ein weiterer Linienkörper (NP) mit Kennwerten (50c - 53c) als Ecken dargestellt wird, dessen Ecken gegenüber dem im ersten stationären Zustand dargestellten Linienkörper eine in Summe gesehen geringstmögliche Abweichung aufweist oder der zweite Linienkörper (P3) der zu den nicht verschobenen Eckpunkten nächstmögliche Linienkörper ist oder eine neue Lösung als Linienkörper dargestellt wird, deren nicht aktiv verschobene Eckpunkte zu den zuvor dargestellten Eckpunkten eine geringste Abweichung besitzen.

25. **Planungswerkzeug** mit einer Datenbank, für eine interaktive Auswahl von Steuergrößen (x₁,y₇, α₁,α₂, α₃, I₁,I₂) eines Strahlentherapieplans aus der Datenbank (1) mit einer Vielzahl von vorberechneten oder vorgegebenen Lösungen,
wobei jede Lösung
(i) einen Strahlentherapieplan repräsentiert, der in technischer Hinsicht selbst aus einer Vielzahl von Steuergrößen oder Befehlen besteht, die einem Strahlentherapiegerät (10,11,12) beaufschlagbar sind (6,7) oder für dieses Gerät (10) zur Verfügung gestellt werden;
(ii) ein Kennwert einer Strahlendosis für ein Zielvolumen oder Target (T) und mindestens zwei vergleichbare Kennwerte für mindestens zwei Risikovolumen (h₁,h₂,h₃) enthält, welche Kennwerte in der Datenbank (1) gespeichert sind;
wobei die Kennwerte (50,51,52,53) für zumindest eine Vielzahl der gespeicherten Lösungen so jeweils zugehörigen nicht übereinander fallenden oder nicht aufeinander liegenden Achsen zuweisbar sind, dass für jede von zumindest zwei Risikoachsen (31,32,33) und die Targetachse (30) jeweils ein Akzeptanzintervall (a₃₀,a₃₁,a₃₂,a₃₃) entsteht, die für alle Achsen gemeinsam eine Planungszone (40;41,42) festlegen.

26. Planungswerkzeug nach Anspruch 25, zur visuell wahrnehmbaren Steuerung einer Auswahl zumindest eines Abschnitts einer Strahlentherapie aus einer Datenbank von mehreren möglichen, insbesondere pareto-optimalen Lösungen, wobei jede der Lösungen eine Vielzahl von Steuereinstellungen des Bestrahlungsgerätes (10,11,12) umfasst, wobei
(i) die mehreren Strahlendosisskalen (30 - 33) von dem Target und den mehreren Risikoorganen in einem Strahlenstern aufgetragen und bildlich dargestellt werden;
(ii) aus den vorab in der Datenbank (1,1a) gespeicherten möglichen Lösungen ein Akzeptanzintervall (a₃₀,a₃₃) auf jedem der Strahlen des Strahlensterns aufgetragen wird, wobei jedes Akzeptanzintervall einen oberen und einen unteren Randwert (30m,30n) aufweist;
(iii) durch eine direkte Verbindung der Randwerte von jeweils auf Nachbarskalen bzw. Nachbarstrahlen liegenden Akzeptanzintervallen ein globaler Planungshorizont als Planungszone (40;41,42) eingerahmt wird;
zur erfassbaren Visualisierung einer in der Datenbank verfügbaren Lösungsvielfalt.

27. Planungswerkzeug nach Anspruch 26, **dadurch gekennzeichnet, dass** Pfeilspitzen an den Strahlen des Sterns in Richtung einer jeweils zunehmenden Dosis bei Targets und in Richtung einer abnehmenden Dosis bei Risiken (h₁,h₂) zeigen.

28. Planungswerkzeug nach Anspruch 26, wobei die Dosiswerte nur einer Lösung aus der Datenbank (1) in den entsprechenden Akzeptanzintervallen als Punkte kenntlich gemacht sind und im wesentlichen gradlinig verbunden sind, um ein Navigations-Polygon (NP) zu bilden, das eine Lösung aus der Datenbank zusammenhängend anschaulich darstellt.

29. Planungswerkzeug nach Anspruch 26, **dadurch gekennzeichnet, dass** nach einem Ziehen mit einem Zeiger eines Bediengerätes (4) an einer Stelle des Navigations-Polygons, insbesondere einer Ecke des Polygons längs des zugehörigen Strahls (33) und innerhalb des entsprechenden Akzeptanzintervalls (a₃₃) das aktuelle Navigations-Polygon (P2) ausgeblendet und anschließend dasjenige Navigations-Polygon (P3) eingeblendet wird, das eine Lösung in der Datenbank visualisiert, deren Bewertung im aktuellen Akzeptanzintervall dem verlagerten Punkt des Navigations-Polygons am nächsten kommt, insbesondere deren Kennwerte in den anderen Akzeptanzintervallen gleichzeitig eine geringstmögliche Abweichung zu den bisher dargestellten Punkten auf den Strahlen des vorhergehenden Navigations-Polygons aufweisen.

30. Planungswerkzeug nach einem der Ansprüche 26 oder 29, **dadurch gekennzeichnet, dass** Markierungselemente (33a,31 a) den Strahlen des Strahlenstemes mit den Strahlendosisskalen zugeordnet sind, um ein Sperren eines jeweiligen Abschnitts (a₃₃) des Akzeptanzintervalls des zugeordneten Strahls (33) zu verursachen, und zwar von dem jeweils schlechtwertigsten Randpunkt bis zu dem angezeigten Eckpunkt des Navigations-Polygons.

31. Planungswerkzeug nach Anspruch 26, **dadurch gekennzeichnet, dass** durch ein Anklicken oder ein Betätigen eines Markierungselements (33a,31a) ein Abschnitt des entsprechenden Akzeptanzintervalls (a₃₃) gesperrt wird oder sperrbar ist.

32. Planungswerkzeug nach Anspruch 31, wobei nach dem Sperren nur noch solche Lösungen aus der Datenbank in einer reduzierten Planungszone (42) selektierbar sind, die außerhalb des gesperrten Abschnitts (41) der Planungszone liegen und zumindest ein Randwert (30n,30m) eines der Akzeptanzintervalle der anderen der Dosisskalen geändert wird.

33. Planungswerkzeug nach Anspruch 32, wobei die eingeschränkte Planungszone (42) als ein Planungshorizontanteil hervorgehoben dargestellt wird, gegenüber dem ausgeblendeten ersten Planungshorizontanteil (41), wobei die beiden Anteile summiert die Planungszone ergeben.

34. Planungswerkzeug nach Anspruch 33, wobei mehrere Abschnitte von mehreren Achsen sperrbar sind, um mehrere Abschnitte des Planungshorizonts zu sperren, unter Belassung eines jeweils weiter reduzierten Rest-Planungshorizontanteils.

35. Planungswerkzeug nach einem der Ansprüche 30 bis 34, wobei durch Betätigen, insbesondere ein Anklicken mit einem Mauszeiger, eines vorher bereits betätigten Markierungselements (33a) der zugehörig gesperrte Abschnitt des Akzeptanzintervals bzw. Planungshorizonts wieder freigegeben wird, um die zugehörigen Lösungen aus der Datenbank wieder selektieren zu können und deren Visualisierungen als zum aktuellen Planungshorizont hinzugenommen dargestellt werden.

36. Planungswerkzeug nach Anspruch 28, wobei ein Übergang von einem Navigations-Polygon (NP=P2) zu einem folgenden Navigations-Polygon (P3) fließend geschieht, mit Darstellung von Zwischenzuständen.

37. Planungswerkzeug nach Anspruch 26, wobei ein Navigations-Polygon vorgesehen ist, das innerhalb der Planungszone sichtbar unterschiedlich dargestellt ist und als Dosisverteilungs-Polygon eine Verteilung von Strahlendosen, insbesondere nach EUD-Werten für Risikoorgane bzw. Target (T,h1,h2,h3) angibt.

## Claims

1. Process for control via a visually perceptible display and for interactive selection of control parameters (x₁, y₇, α₁, α₂, α₃, I₁, I₂) of a radiation treatment plan from a database (1) having a plurality of pre-calculated or preset solutions, wherein each solution
(i) represents a radiation treatment plan, which in the technical respect itself consists of a plurality of control parameters or commands, which can be exposed (6, 7) to a radiation treatment apparatus (10, 11, 12) or are provided for this apparatus (10), temporally before a possible execution of control parameters or commands within the framework of carrying out a treatment;
(ii) contains characteristics of radiation doses for a target volume or target (T) and at least one risk volume (h₁, h₂, h₃), which are stored in the database (1);
and wherein
(a) several axes are plotted or displayed visibly on a display device (3; 3a, 3b) as radiation dose scales (30, 31, 32, 33) for the target volume (T) and the at least one risk volume (h₁, h₂, h₃), to form at least one risk axis and target axis, wherein no axis coincides with a further axis and adjacent axes (30, 32; 30, 33) do not run parallel;
(b) the characteristics (50, 51, 52, 53) of the radiation doses for at least a plurality of the stored solutions are assigned to the particular associated axes, so that for each risk axis (31, 32, 33) and the target axis (30), an acceptance interval (a₃₀, a₃₁, a₃₂, a₃₃) is produced which define a planning zone (40; 41, 42) for all axes together;
(c) the planning zone (4) is emphasised on the display device (3) with respect to the environment (45, 45a) or is displayed visibly in a manner capable of differentiation with respect to the environment.

2. Process according to claim 1, wherein each axis (30 - 33) has a section as an acceptance interval, which lies within the planning zone (40) and each section has an upper and a lower boundary value (30m, 30n).

3. Process according to claim 1 or 2, wherein the planning zone has as a planning area, a polygonal shape with inner and outer boundary polygon (Pᵢ, Pₐ).

4. Process according to claim 1, wherein connecting paths of interval ends or the boundary values according to claim 2 of adjacent axes (30, 32) define an outer and an inner limit of the planning zone for recordable visualisation of the solution variety stored in the database.

5. Process according to claim 1, wherein within the planning zone in the state of a first stationary display, only one solution from the database is displayed, so that its characteristics (50, 51, 52, 53; 50b - 53b) of radiation doses on the axes (30 - 33) are connected by visible lines to form a navigation line body (NP, P1, P2) as a polygon.

6. Process according to claim 5, wherein all connecting lines lie within the planning zone (40).

7. Process according to claim 5 or 6, wherein the connecting lines run in a straight line in order to form the polygon (P1) as a navigation line body with a comer number equal to a number of outer comers (Pa, Pa') of the planning zone (40).

8. Process according to claim 5, wherein in a transitional state from the first stationary display to a second stationary display (50c - 53c), a shape or position of the first line body (NP) is changed and a second line body is formed (P3), but remains within the planning zone (40), to display a second solution from the database with its associated characteristics for radiation doses of target volumes and risk volumes.

9. Process according to claim 8, wherein during the transitional state, a change from the first line body (P2) to the second line body (P3) takes place fluidly or gradually, in order to make the change clear and visible, and in the visibly fluid change, two solutions or associated line bodies (P3, P2) are visible at the same time in each case at least in sections.

10. Process according to claim 1, wherein each solution stands for a number of dose distributions for several angular positions (α₁ - α₅), for indirect transfer to a radiation head (12) of a treatment apparatus (10, 11), in particular to associated multiple strips (13, 14) of a multi-leaf collimator upstream of a radiation head (12).

11. Process according to claim 1, wherein only one single solution of the plurality of stored solutions is displayed in the planning zone (40), which is set off from the background (45a, 45), in particular by colour or by the grey value, during a state of a stationary display.

12. Process according to claim 1, wherein a first section (41) of the planning zone is blocked or can be blocked, in order to leave the remaining planning zone (42) for changes in a navigation body (NP) inscribed in the planning zone (40).

13. Process according to claim 12, wherein blocking can be removed again, in order to add again the first section (41) to the remaining section for changes in the inscribed navigation body.

14. Process according to claim 12 or 13, wherein blocking of the first section is effected by blocking a characteristic (53) on one of the axes (33), as a result of which the characteristic (53) becomes a peak and a new comer of a new outer polygon (Pₐ') is formed and certain solutions of the database are blocked
(i) for a display as a navigation line body (NP) and
(ii) for a selection,
wherein the certain solutions have a characteristic as dose characteristic on the one axis (33) which lies in the blocked section (41).

15. Process according to claim 1, wherein the characteristics (50, 51, 52, 53) of the radiation doses are represented by EUD values as averaged dose distributions per organ or target.

16. Process according to claim 5, wherein by actuating a displayed defined control section (75), storage of a number of the currently displayed solution is initiated in the current navigation line body (P1).

17. Process according to claim 16, wherein by actuating a further defined control section (75) of the display, switching into a visible mode is carried out, in order to provide from the database (1) the solutions to the stored numbers for display (3, 3a) which have been stored using the first control section of the viewing screen (3).

18. Process according to claim 17, wherein with the aid of recorder sections (70) on a viewing screen as display device, the visualisations corresponding to the associated stored solutions are displayed according to a user request.

19. Process according to claim 5, wherein solutions, in particular pointers to stored data records of the database, which solutions are assigned to a currently displayed navigation line body (P2), are stored (5, 1) sequentially in a background automatically in a log data file when actuating a control section.

20. Process according to claim 19, wherein actuation of one of several recorder image sections (70), the functions of which correspond to recorder functions, the visualisations of the solutions referenced in the log data file are called up according to user requests.

21. Process according to claim 20, wherein actuation of a control section already actuated previously triggers switching to a mode which permits continuation of navigation in the current planning zone.

22. Process according to one of claims 19 to 21, in which actuation is clicking using a mouse pointer or contacting the viewing screen at a specific point as viewing screen (3) as display device.

23. Process according to claim 12, wherein blocking of a first section (41) is effected by blocking a more inferior scale value, based on dose and dose direction on a particular axis (30 - 33).

24. Process according to claim 5, wherein in a second stationary display, a further line body (NP) with characteristics (50c - 53c) is displayed as comers, the comers of which have the lowest possible deviation seen in total with respect to the line body displayed in the first stationary state, or the second line body (P3) is the line body closest to the non-displaced comer points, or a new solution is displayed as a line body, the non-actively displaced comer points of which have the lowest deviation from the previously displayed comer points.

25. Planning tool having a database for interactive selection of control parameters (x₁, y₇, α₁, α₂, α₃, I₁, I₂) of a radiation treatment plan from the database (1) having a plurality of pre-calculated or preset solutions, wherein each solution
(i) represents a radiation treatment plan, which in the technical respect itself consists of a plurality of control parameters or commands, which can be exposed (6, 7) to a radiation treatment apparatus (10, 11, 12) or are provided for this apparatus (10);
(ii) contains a characteristic of a radiation dose for a target volume or target (T) and at least two comparable characteristics for at least two risk volumes (h₁, h₂, h₃), which characteristics are stored in the database (1);
wherein the characteristics (50, 51, 52, 53) for at least a plurality of the stored solutions can be assigned in each case to associated non-coincident axes or axes not lying one on another, so that for each of at least two risk axes (31, 32, 33) and the target axis (30), in each case an acceptance interval (a₃₀, a₃₁, a₃₂, a₃₃) is produced which define a planning zone (40; 41, 42) for all axes together.

26. Planning tool according to claim 25, for visually perceptible control of a selection of at least one section of a radiation treatment from a database of several possible, in particular pareto-optimal solutions, wherein each of the solutions comprises a plurality of control settings of the radiation apparatus (10, 11, 12), wherein
(i) the several radiation dose scales (30 - 33) of the target and the several risk organs are plotted on a ray star and displayed pictorially;
(ii) from the possible solutions stored to start with in the database (1, 1a), an acceptance interval (a₃₀, a₃₃) is plotted on each of the rays of the ray star, wherein each acceptance interval has an upper and a lower boundary value (30m, 30n);
(iii) by direct connection of the boundary values of acceptance intervals lying in each case in neighbouring scales or neighbouring rays, a global planning horizon is framed as a planning zone (40; 41, 42);
for recordable visualisation of a solution variety available in the database.

27. Planning tool according to claim 26, **characterised in that** arrow tips on the rays of the star point in the direction of an in each case increasing dose for targets and in the direction of a decreasing dose for risks (h₁, h₂).

28. Planning tool according to claim 26, wherein the dose values of only one solution from the database (1) are made known in the corresponding acceptance intervals as points and are connected essentially in a straight line in order to form a navigation polygon (NP), which clearly displays a solution from the database in a coherent manner.

29. Planning tool according to claim 26, **characterised in that** after pulling using a pointer of an operating apparatus (4) at a point of the navigation polygon, in particular a comer of the polygon along the associated ray (33) and within the corresponding acceptance interval (a₃₃), the current navigation polygon (P2) is screened out and then that navigation polygon (P3) is faded in, which visualises a solution in the database, the evaluation of which in the current acceptance interval comes closest to the displaced point of the navigation polygon, in particular the characteristics of which in the other acceptance intervals have at the same time a lowest possible deviation from the hitherto displayed points on the rays of the preceding navigation polygon.

30. Planning tool according to one of claims 26 or 29, **characterised in that** marking elements (33a, 31a) are assigned to the rays of the ray star with the radiation dose scales, in order to cause blocking of a particular section (a₃₃) of the acceptance interval of the assigned ray (33), and specifically by the in each case most inferior value boundary point up to the indicated comer point of the navigation polygon.

31. Planning tool according to claim 26, **characterised in that** by clicking or actuating a marking element (33a, 31a), a section of the corresponding acceptance interval (a₃₃) is blocked or can be blocked.

32. Planning tool according to claim 31, wherein after blocking, only those solutions from the database in a reduced planning zone (42) can be selected, which lie outside of the blocked section (41) of the planning zone and at least one boundary value (30n, 30m) of one of the acceptance intervals of the other of the dose scales is changed.

33. Planning tool according to claim 32, wherein the restricted planning zone (42) is displayed emphasised as a planning horizon portion, compared to the screened-out first planning horizon portion (41), wherein the two portions added up produce the planning zone.

34. Planning tool according to claim 33, wherein several sections of several axes can be blocked, in order to block several sections of the planning horizon, while leaving an in each case further reduced residual planning horizon portion.

35. Planning tool according to one of claims 30 to 34, wherein by actuation, in particular clicking using a mouse pointer, a previously already actuated marking element (33a), the associated blocked section of the acceptance interval or planning horizon is released again, in order to be able to select the associated solutions from the database again and the visualisations of which are displayed as being added to the current planning horizon.

36. Planning tool according to claim 28, wherein a transition from one navigation polygon (NP=P2) to a following navigation polygon (P3) takes place fluidly, with display of intermediate states.

37. Planning tool according to claim 26, wherein a navigation polygon is provided, which is displayed to be visibly different within the planning zone, and indicates as a dose distribution polygon, a distribution of radiation doses, in particular according to EUD values for risk organs or target (T, h1, h2, h3).

## Revendications

1. **Procédé** de commande par représentation visuellement appréhendable et de sélection interactive de grandeurs de commande (x₁, y₇, α₁, α₂, α₃, I₁, I₂) d'un plan de radiothérapie d'une banque de données (1) ayant une pluralité de solutions précalculées et prédéterminées,
**chaque solution**
(i) représentant un plan de radiothérapie, constitué, du point de vue technique proprement dit, d'une pluralité de grandeurs de commande ou d'instructions qui peuvent être introduites (6,7) dans un appareil de radiothérapie (10,11,12), ou qui peuvent être disponibles pour cet appareil (10), sur le plan du temps avant une éventuelle exécution des grandeurs de commande ou des instructions dans le cadre de la mise en oeuvre d'une thérapie;
(ii) contenant des caractéristiques de doses d'irradiation pour un volume cible ou Target (T) et au moins un volume de risque (h₁,h₂,h₃), stockées dans une banque de données (1);
et dans lequel
(a) plusieurs axes sont visiblement portés ou représentés comme des échelles de doses d'irradiation (30,31,32,33) pour le volume cible (T) et au moins un volume de risque (h₁, h₂, h₃), sur un dispositif de représentation (3;3a,3b)pour la formation d'au moins un axe de risque et d'un axe Target, aucun axe ne se superposant à un autre, ni n'évoluant parallèlement aux axes voisins (30,32;30,33);
(b) les caractéristiques (50,51,52,53) des doses d'irradiation pour au moins une pluralité des solutions stockées sont attribuées ainsi à chacun des axes respectifs pour que pour chaque axe de risque (31,32,33) et l'axe Target (30) apparaisse un intervalle d'admission (a₃₀,a₃₁,a₃₂,a₃₃), lesquels fixent ensemble, pour tous les axes, une zone de programmation (40;41,42);
(c) la zone de programmation (40) sur le dispositif de représentation (3) est, face au champ périphérique (45,45a), représentée élevée ou est représentée face au champ périphérique de manière visible et distinctive.

2. Procédé selon la revendication 1, dans lequel chaque axe (30-33) présente une section, sous forme d'un intervalle d'admission, qui est situé au sein de la zone de programmation (40), et chaque section présente une valeur frontière supérieure et une inférieure (30m,30n).

3. Procédé selon la revendication 1 ou 2, dans lequel la zone de programmation, comme surface de programmation, présente une configuration polygonale, avec un polygone de bord (Pᵢ,Pₐ) intérieur et extérieur.

4. Procédé selon la revendication 1, dans lequel des droites de liaison des extrémités d'intervalles ou des valeurs de bord selon la revendication 2 d'axes voisins (30,32) fixent une limite extérieure et une intérieure de la zone de programmation, pour visualiser de manière détectable la pluralité de solutions stockées dans la banque de données.

5. Procédé selon la revendication 1, dans lequel, au sein de la zone de programmation, dans une première représentation stationnaire, seule une solution de la banque de données est représentée de telle sorte que ses caractéristiques (50,51,52,53;50b-53b) de doses d'irradiation sur les axes (30-33) sont reliées par des lignes visibles, pour former un corps linéaire de navigation (NP,P1,P2) sous forme de polygone.

6. Procédé selon la revendication 5, dans lequel toutes les lignes de liaison sont situées au sein de la zone de programmation (40).

7. Procédé selon la revendication 5 ou 6, dans lequel les lignes de liaison évoluent en ligne droite pour former le polygone (P1) comme corps linéaire de navigation avec un nombre égal d'angles, comme un nombre d'angles extérieurs (Pa, Pa') de la zone de programmation (40).

8. Procédé selon la revendication 5, dans lequel, **en transition** de la première représentation stationnaire vers une **deuxième représentation stationnaire** (50c-53c), une forme ou la situation du premier corps linéaire (NP) est modifiée et un deuxième corps linéaire se forme (P3), mais reste au sein de la zone de programmation (40), pour représenter une deuxième solution de la banque de données avec ses caractéristiques respectives de doses d'irradiation de volume cible et de volume de risque.

9. Procédé selon la revendication 8, dans lequel, pendant la transition, un échange du premier corps linéaire (P2) en le deuxième corps linéaire (P3) s'effectue sans heurts ou de manière progressive pour rendre l'échange explicite et visible, et pour rendre visible en même temps, lors de l'échange visiblement sans heurts, deux solutions ou des corps linéaires relatifs (P3,P2), chaque fois au moins par section.

10. Procédé selon la revendication 1, dans lequel chaque solution représente un nombre de distributions de doses pour plusieurs positions d'angle (α₁-α₅) pour une diffusion non immédiate sur une tête d'irradiation (12) d'un appareil thérapeutique (10,11), en particulier sur des bandes multiples (13,14) respectives d'un collimateur multilame devant une tête d'irradiation (12).

11. Procédé selon la revendication 1, dans lequel, dans la zone de programmation (40) qui commence de l'arrière-plan (45a,45) en particulier en couleurs ou de la valeur de gris, pendant une représentation stationnaire, une seule de la pluralité des solutions stockées est représentée.

12. Procédé selon la revendication 1, dans lequel une première section (41) de la zone de programmation est bloquée ou blocable pour libérer la zone de programmation restante (42) en vue de modifications d'un corps de navigation (NP) inscrit dans la zone de programmation (40).

13. Procédé selon la revendication 12, dans lequel le blocage peut être à nouveau levé pour ajouter à nouveau la première section (41) à la section restante en vue de modifications du corps de navigation inscrit.

14. Procédé selon la revendication 12 ou 13, dans lequel le blocage de la première section est le fait du blocage d'une caractéristique (53) sur un des axes (33), ce qui fait que la caractéristique (53) devient un point dominant et forme l'angle nouveau d'un nouveau polygone extérieur (Pₐ') et que certaines solutions de la banque de données sont bloquées
(i) pour une représentation sous forme de corps linéaire de navigation (NP) et
(ii) pour une sélection,
où les différentes solutions possèdent une caractéristique comme une caractéristique de dose sur un axe (33) situé dans la section bloquée (41).

15. Procédé selon la revendication 1, dans lequel les caractéristiques (50,51,52,53) des doses d'irradiation sont représentées par des valeurs EUD comme des distributions de doses moyennes par organe ou Target.

16. Procédé selon la revendication 5, dans lequel le stockage d'un numéro de la solution actuellement représentée dans le corps linéaire de navigation actuel (P1) est initié par l'actionnement d'une section de commande (75) limitée représentée.

17. Procédé selon la revendication 16, dans lequel la commutation en mode affichage s'effectue par l'actionnement d'une autre section de commande (75) limitée de la représentation pour mettre, à partir de la banque de données (1), les solutions à disposition en numéros stockés en vue de la représentation (3,3a), stockés en utilisant la première section de commande de l'écran d'affichage (3).

18. Procédé selon la revendication 17, dans lequel les visualisations correspondant aux solutions stockées respectives sont représentées, en fonction du souhait de l'utilisateur, à l'aide de sections d'enregistrement (70) sur un écran d'affichage faisant office de dispositif de représentation.

19. Procédé selon la revendication 5, dans lequel, sur l'actionnement d'une section de commande, les solutions, en particulier les pointeurs sur des enregistrements stockés de la banque de données, lesquelles solutions sont attribuées à un corps linéaire de navigation (P2) actuellement représenté, sont stockées (5,1) séquentiellement dans un arrière-plan de manière automatique dans un fichier journal.

20. Procédé selon la revendication 19, dans lequel l'actionnement d'une de plusieurs sections d'image d'enregistrement (70), dont les fonctions correspondent à des fonctions d'enregistrement, ainsi que les visualisations des solutions référencées dans le fichier journal sont extraites en fonction du souhait de l'utilisateur.

21. Procédé selon la revendication 20, dans lequel l'actionnement d'une section de commande précédemment actionnée déclenche une commutation en un mode qui permet la poursuite de la navigation dans la zone actuelle de programmation.

22. Procédé selon l'une des revendications 19 à 21, dans lequel un actionnement est un clic à l'aide d'une souris ou un contact avec l'écran d'affichage sur un endroit spécifique de l'écran d'affichage (3) comme dispositif de représentation.

23. Procédé selon la revendication 12, dans lequel le blocage d'une première section (41) s'effectue par un blocage d'une valeur d'échelle plus médiocre, sur base de la dose et du sens de la dose sur un axe respectif (30-33).

24. Procédé selon la revendication 5, dans lequel dans une deuxième représentation stationnaire, un autre corps linéaire (NP) portant des caractéristiques (50c-53c) est représenté sous forme de coins dont les angles par rapport au corps linéaire représenté dans un premier état stationnaire, présente une déviation qui, considérée dans sa somme, est la plus petite possible ou le deuxième corps linéaire (P3) est le corps linéaire le plus proche par rapport aux sommets non décalés, ou une nouvelle solution est représentée comme un corps linéaire dont les sommets non activement décalés par rapport aux sommets précédemment représentés, présentent la plus petite déviation.

25. **Outil de programmation** ayant une banque de données, prévu pour une sélection interactive de grandeurs de commande (x₁, y₇, α₁, α₂, α₃, I₁, I₂) d'un plan de radiothérapie de la banque de données (1) présentant une pluralité de solutions précalculées ou prédéterminées,
**chaque solution**
(i) représentant un plan de radiothérapie, constitué, du point de vue technique proprement dit, d'une pluralité de grandeurs de commande ou d'instructions qui peuvent être introduites (6,7) dans un appareil de radiothérapie (10,11,12), ou qui peuvent être disponibles pour cet appareil (10);
(ii) contenant une caractéristique de doses d'irradiation pour un volume cible ou Target (T) et au moins deux caractéristiques comparables pour au moins deux volumes de risque (h₁, h₂, h₃), lesquelles caractéristiques sont stockées dans la banque de données (1);
dans lequel les caractéristiques (50,51,52,53) pour au moins une pluralité des solutions stockées sont attribuables ainsi à chacun des axes respectifs ne tombant ni ne se chevauchant l'un sur l'autre pour que pour chacun d'au moins deux axes de risque (31,32,33) et l'axe Target (30) apparaisse chaque fois un intervalle d'admission (a₃₀, a₃₁, a₃₂, a₃₃), lesquels fixent ensemble, pour tous les axes, une zone de programmation (40;41,42).

26. Outil de programmation selon la revendication 25, pour une commande visuellement appréhendable d'une sélection d'au moins une section d'une radiothérapie d'une banque de données de plusieurs solutions possibles, en particulier paréto-optimales, chacune des solutions comprenant une pluralité de paramètres de commande de l'appareil d'irradiation (10,11,12), dans lequel
(i) les différentes échelles de doses d'irradiation (30-33) du Target et des différents organes de risque sont portées dans une étoile de rayons et représentées en image;
(ii) des solutions possibles précédemment stockées dans la banque de données (1,1a), un intervalle d'admission (a₃₀, a₃₃) est porté sur chacun des rayons de l'étoile de rayons, chaque intervalle d'admission présentant une valeur frontière supérieure et une inférieure (30m, 30n) ;
(iii) par une liaison directe des valeurs frontières des intervalles d'admission situés chaque fois sur des échelles voisines ou, selon le cas, rayons voisins, un horizon de programmation global se trouve encadré comme une zone de programmation (40;41,42);
pour une visualisation appréhendable d'une pluralité de solutions disponibles dans la banque de données.

27. Outil de programmation selon la revendication 26, **caractérisé en ce que** des pointes de flèches sur les rayons de l'étoile indiquent une dose respectivement en augmentation pour des Targets et indiquent une dose en diminution pour des risques (h₁,h₂).

28. Outil de programmation selon la revendication 26, dans lequel les valeurs de dose d'une seule solution de la banque de données (1) se reconnaissent dans les intervalles correspondants d'admission sous forme de points et sont liées essentiellement en ligne droite pour former un polygone de navigation (NP) qui représente explicitement en continu une solution de la banque de données.

29. Outil de programmation selon la revendication 26, **caractérisé en ce qu'**après un glissement avec le pointeur d'un appareil de commande (4) à un endroit du polygone de navigation, en particulier d'un angle du polygone le long du rayon relatif (33) et dans les limites de l'intervalle correspondant d'admission (a₃₃), le polygone de navigation actuel (P2) s'éteint et ensuite l'autre polygone de navigation (P3) s'illumine, de sorte qu'une solution de la banque de données se visualise, dont l'évaluation dans l'intervalle actuel d'admission se rapproche le plus du point translaté du polygone de navigation, en particulier dont les caractéristiques dans les autres intervalles d'admission présentent simultanément la plus petite déviation possible par rapport aux points jusqu'à présent représentés sur les rayons du polygone de navigation précédent.

30. Outil de programmation selon l'une des revendications 26 ou 29, **caractérisé en ce que** des éléments de marquage (33a,31a) sont attribués aux rayons de l'étoile de rayons avec les échelles de doses d'irradiation, pour provoquer un blocage d'une section respective (a₃₃) de l'angle d'admission du rayon attribué (33) et ce, du point de bord à la plus médiocre valeur jusqu'au point d'angle indiqué du polygone de navigation.

31. Outil de programmation selon la revendication 26, **caractérisé en ce que** par un clic ou l'actionnement d'un élément de marquage (33a,31a), une section de l'intervalle correspondant d'admission (a₃₃) est bloqué ou blocable.

32. Outil de programmation selon la revendication 31, dans lequel après le blocage, seules des solutions de la banque de données d'une zone de programmation réduite (42) peuvent être sélectionnées, qui se situent à l'extérieur de la section bloquée (41) de la zone de programmation et où ne change qu'une valeur de bord (30n,30m) d'un des angles d'admission de l'autre des échelles de doses.

33. Outil de programmation selon la revendication 32, dans lequel la zone de programmation réduite (42) est représentée élevée comme une partie horizontale de programmation, en face de la première partie horizontale de programmation (41), où les deux parties donnent, en somme, la zone de programmation.

34. Outil de programmation selon la revendication 33, dans lequel plusieurs sections de plusieurs axes sont blocables pour bloquer plusieurs sections de l'horizon de programmation, en libérant une partie de l'horizon de programmation résiduelle chaque fois encore réduite.

35. Outil de programmation selon l'une des revendications 30 à 34, dans lequel l'actionnement, en particulier le clic d'une souris, d'un élément de marquage (33a) précédemment déjà actionné libère la section relative bloquée de l'intervalle d'admission ou, selon le cas, horizon de programmation, pour pouvoir à nouveau sélectionner les solutions relatives de la banque de données et leurs visualisations sont représentées ajoutées à l'horizon actuel de programmation.

36. Outil de programmation selon la revendication 28, dans lequel une transition d'un polygone de navigation (NP=P2) vers un polygone de navigation (P3) qui suit s'effectue sans heurts, avec représentation des états intermédiaires.

37. Outil de programmation selon la revendication 26 qui prévoit un polygone de navigation représenté visiblement distinguable au sein de la zone de programmation et donne à titre de polygone de distribution des doses une distribution des doses d'irradiation, en particulier selon les valeurs EUD pour des organes de risque ou, selon le cas, Target (T, h₁, h₂, h₃) .
